(19) Europäisches Patentamt
European Patent Office
Office européen des brevets

(11) EP 2 823 804 B1

(12) **EUROPEAN PATENT SPECIFICATION**

(45) Date of publication and mention of the grant of the patent:
**21.08.2019 Bulletin 2019/34**

(51) Int Cl.:
***A61K 8/11*** *(2006.01)* ***A61K 8/34*** *(2006.01)*
***A61K 8/73*** *(2006.01)* ***A61K 8/29*** *(2006.01)*

(21) Application number: **13305997.2**

(22) Date of filing: **12.07.2013**

(54) **Color changing composition comprising pigments and a high amount of water**

Farbveränderliche Zusammensetzung mit Pigmenten und einem hohen Anteil an Wasser

Composition changeant de couleur comprenant des pigments et une grande quantité d'eau

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**14.01.2015 Bulletin 2015/03**

(73) Proprietor: **L'Oréal**
**75008 Paris (FR)**

(72) Inventors:
- **Ricard, Audrey**
  **94210 La Varenne (FR)**
- **El Achkar, Micheline**
  **75020 Paris (FR)**

(74) Representative: **Nony**
**11 rue Saint-Georges**
**75009 Paris (FR)**

(56) References cited:
**EP-A1- 2 277 982**
**WO-A1-2013/108410**
**US-A- 4 756 906**
**US-A1- 2011 229 536**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to a color-changing composition comprising a high amount of water, in particular useful for care, hygiene and/or makeup of keratin materials.

In particular, a color-changing composition according to the invention may be any type of cosmetic composition such as a foundation, a lip balm, a lip gloss, an eyeliner, a mascara, a body makeup product, a skin coloring product, a care product such as a care cream, a 'BB' product (Blemish Balm product able to cover imperfections), a tinted cream or an anti-sun product, preferably a foundation or BB product. The color-changing composition according to the invention may be liquid or solid, in particular liquid.

A composition of the invention is especially a composition intended to be applied to a keratin material, in particular the skin and more particularly facial skin.

Cosmetic compositions, especially foundations, are commonly used to give the skin an aesthetic color, but also to hide skin imperfections such as redness and/or marks. In this regard, many formulations have been developed to date.

In this respect, there is a growing interest in cosmetic products that provide a change in color in response to external incentives such for example shear force.

Generally, this purpose is achieved by including in cosmetic composition microencapsulated colorants wherein, upon application on the skin, the composition provides the expected changing color. More particularly, the change of color is provided by the colorant-containing microcapsules, which upon rupture by application of a mechanical force, release the entrapped colorant into the composition, thereby changing its color. A mechanical action such as rubbing spread the topical composition and facilitates its penetration into the skin. The immediate change of color of the composition provides a visual esthetical effect.

Different types of entrapped colorants and more particularly pigments-containing microcapsules are already available. They mainly differ through the type of entrapping material(s) and/or the type of encapsulation.

However, colorant-containing microcapsules are not always stable in any media.

With some colorant-containing microcapsules it may be difficult to permanently retain the colorant over long periods of time and when subjected to different environments and conditions. This is true of pigments, oil soluble dyes, and water soluble dyes. Thus, such microcapsules may gradually release the colorant, or "bleed", over time when tested for prolonged periods at elevated temperatures. Color bleed occurs when a dye or pigment migrates through or off of microspheres/microcapsules through contact with moisture and/or other ingredients in a formulation such as alcohols or glycols, surfactants, silicones, oils, preservatives, salts and other components typically found in cosmetic formulations. Leeching or bleed of the colorant in cosmetic composition can impair the long term visual effect of the cosmetic both in the container and on the substrate.

Furthermore, some pigment-containing microcapsules may have some stability issues depending on the cosmetic composition and associated solvents/ingredients.

In this case, rupture of the microcapsules spontaneously occurs in the bulk of the emulsion without that any rubbing or pressing force has been applied. This rupture of the microcapsules often results from a softening of the external layer and leads to a release of the color e.g. pigments in the emulsion bulk.

As a consequence the bulk appearance becomes dirty grey, and messed up if those kinds of breakable microcapsules are introduced in formula. The release of the microcapsules leads to visible effects like emergence of colored bead in the white bulk and also coloration of the bulk.

It is known in the art to introduce pigments into cosmetic compositions, especially compositions for care or for making-up comprising a high amount of water. Theses compositions are expected to have a fresh sensorial effect on application, and to confer an appropriate color shade to the skin, furthermore their ability to include high amounts of active ingredients is highly appreciated. US2011/229536 discloses a color-changing composition comprising encapsulated active agents and colorants, wherein the composition changes color upon application, indicating thus the delivery of active agents from said composition onto the skin.

Such fresh sensorial and optical effects are for example needed in formulations of the "quick break type", in face serums, in eye serums and in foundations.

However, the introduction of a high amount of pigments may impair the fresh effect: the pigments tend to absorb water leading the composition to dry out more rapidly, thus rendering the composition more difficult to apply uniformly.

As a consequence, the color of such a composition after application to the skin does not reach the expected requirement regarding uniformity. Moreover it is not always possible to remedy this issue once the composition has formed a dry film on the skin.

Thus, there remains a need for color-changing compositions comprising a high amount of water and conferring an appropriate color to the skin as well as a fresh effect.

It is particularly desirable to have such compositions which are easy to apply and which permit a homogenous color deposition.

Thus, there is a need to obtain color-changing compositions comprising a high amount of water and conferring

required color properties, these compositions advantageously comprising neither a too high amount of pigments nor high quantities of homogenizing fillers.

Particularly it remains a need to have such compositions which remain stable over a prolonged time e.g. during 2 months at room temperature and even at 37°C or 45°C.

There remains a need to have color-changing compositions wherein the number of microcapsules which break, without applying a force able to achieve this rupture of the microcapsules, during the storage is very low that means less than 5%. The force able to achieve the microcapsule rupture is the minimal conventional force necessary for applying or spreading a cosmetic composition on the skin.

It also remains a need to propose a composition with caring appearance which provides good makeup effects, particularly a good covering effect.

Based on this, lots of cosmetic companies focus on looking for some pigments encapsulating technologies, aiming to get clear and clean bulk tone, but still delivering proper makeup results in particular a final glowing and natural look.

Thus there is a need of colorant-containing microcapsules, which capsules retain good shatter resistance and exhibit improved bleed resistance.

There is also a need to provide a cosmetic composition which allows the preferred coloration or gradation pattern to be adjusted by varying the method or intensity of application onto the skin or the use of microcapsules containing different colorants.

There is also a need to provide a cosmetic composition stable with a large panel of solvent/ingredient associated.

There is also a need to provide a cosmetic composition wherein the microcapsules are or are not visible inside the bulk of the composition depending on the desired appearance.

There is also a need for a cosmetic composition containing pigment-encapsulated microcapsules which do not provoke to the user a discomfort feeling when applied.

There is also a need to provide a cosmetic composition containing pigment-encapsulated microcapsules which disintegrate rapidly indeed immediately when applied, with a liquid or fresh feeling on the skin and leading to colored compositions devoid of any granular aspect. Particularly, the composition may present different shades or color gradations depending on the rubbing strength.

There is also a need to provide pigment-encapsulated microcapsules with a hardness sufficient to be compounded in an industrial process without alteration. Advantageously the hardness of the microcapsules does not significantly decrease during the preparation process.

In particular, the technical problem underlying the present invention as described above, which lies notably in obtaining cosmetic compositions comprising a high amount of water and conferring required color properties has been solved by using microcapsules containing releasable colorant(s) in association with conventional pigments.

Surprisingly and advantageously, the compositions according to the invention meet the needs of the prior art.

The present text describes a color-changing composition for caring for and/or making up keratin materials comprising, in a physiologically acceptable medium:

a) at least 50 % by weight of an aqueous phase relative to the weight of the composition,
b) microcapsules containing releasable colorant(s),
c) at least one pigment chosen from organic pigments and non-lamellar mineral pigments,
said pigment and microcapsules being present in the composition in a weight ratio of pigments / microcapsules > 0,5.

Thus, according to one of its aspects, a subject of the invention is a color-changing composition, that is a composition which changes color upon application, for caring for and/or making up keratin materials comprising, in a physiologically acceptable medium:

a) at least 60 % by weight of an aqueous phase relative to the weight of the composition,
b) microcapsules containing releasable colorant(s),
c) at least one pigment chosen from organic pigments and non-lamellar mineral pigments, said pigment being not encapsulated in the microcapsules,
said pigment and microcapsules being present in the composition in a weight ratio of pigments / microcapsules > 1.5,

wherein the microcapsules comprise

- a core comprising one organic material, said core comprising at least one monosaccharide or monosaccharide-polyol advantageously selected from mannitol, erythritol, xylitol, sorbitol and mixtures thereof, preferably mannitol, as said organic material,
- at least one layered coating surrounding said core, the layered coating comprising at least one polymer, at least

one colorant, and advantageously at least one lipid-based material,

wherein said composition comprises
a gelifying agent chosen from polymers comprising 2-acrylamido-2-methylpropanesulphonic acid units.

The composition comprises a physiologically acceptable medium comprising an aqueous phase and optionally a fatty phase.

According to a preferred embodiment, the compositions comprising a fatty phase are emulsions and preferably O/W emulsions.

According to a preferred embodiment, the O/W emulsions according to the invention contain a fatty phase, in particular a liquid fatty phase, in an amount of at least 1% , preferably from 2 to 50%, more preferably from 5 to 45% et advantageously from 7 to 25% weight relative to the weight of said emulsion.

The microcapsules containing releasable colorant(s) used according to the invention comprise:

- a core comprising one organic material,
- at least one layered coating surrounding said core, the layered coating comprising at least one polymer, at least one colorant, and advantageously at least one lipid-based material.

The microcapsules used according to the present invention are breakable upon spreading of the composition over the skin. The rubbing or pressing of the composition on the skin allows the release of the microcapsule content.

The compositions according to the present invention are notably stable, particularly during 2 months at room temperature and even at 37°C or 45°C and they present optimal cosmetic properties. Indeed the compositions according to the present invention present an appropriate fluidity: they are easy to handle and further easy to apply and to spread on the skin. Furthermore, the inventors noticed that the compositions according to the invention stay without any color release.

These compositions also present a required texture for a cosmetic use: they are not sticky, soft to the touch and the texture is rebounded.

Generally, the microcapsules used according to the invention have average particle sizes of up to about 800 μm in diameter. Preferably the average particle size is less than about 400 μm in diameter of the colorant microcapsules for skin care applications. Preferably, the average particle size will be from 10 μm to 800μm, advantageously from 40μm to 800μm, in particular from 50 μm to 600 μm, and in particular from 50μm to 400μm, and in particular from 100 μm to 400μm in diameter.

According to a preferred embodiment, the average particle size is in the range of about 40 μm to 400 μm in diameter, preferably of about 50 μm to 300 μm in diameter, in particular from 60μm to 250μm in diameter and more preferably of about 80 μm to 200 μm in diameter.

Preferably the microcapsules containing releasable colorant(s) are multi-layered microcapsules.

Preferably the microcapsules containing releasable colorant(s) are multi-layered microcapsules containing releasable colorant(s), said microcapsules comprising:

- an uncolored core consisting in one organic material, and
- a multi-layered coating surrounding said core and comprising at least one organic inner layer and one organic outer layer of different color and entrapping respectively at least one colorant

Preferably, the microcapsules comprise at least two layers preferably at least one organic colored inner layer and one organic outer layer of different color.

The core comprises at least one monosaccharide or its derivatives as said organic material, in particular a monosaccharide-polyol advantageously selected from mannitol, erythritol, xylitol, sorbitol and mixtures thereof, preferably mannitol.

Advantageously, the layered coating surrounding said core comprises at least one hydrophilic polymer(s) selected from the group consisting of polysaccharides and derivatives, preferably the ones including one type of ose or several type of ose(s), preferably several type of ose(s) including at least D-glucose units, in particular starch and derivatives, cellulose or derivatives, and more preferably starch and derivatives.

Preferably, the microcapsules include at least one lipid based material, preferably with amphiphilic properties such as lecithins and in particular hydrogenated lecithin.

Advantageously the core represents from 1% to 50% by weight, preferably 5 to 30% by weight, and in particular from 10 to 20% by weight relative to the total weight of the microcapsule.

Advantageously, the colorant(s) represent from 20% to 90%, preferably from 30% to 80%; in particular from 50% to 75% by weight relative to the microcapsule.

Particularly the microcapsules comprises at least:

- a inner core made of monosaccharide-polyol, preferably mannitol,
- at least two layers of different color,
- at least one hydrophilic polymer preferably selected from polysaccharide or derivatives, and more preferably from starch or derivatives,

and advantageously at least one lipid based material, preferably an amphiphilic compound, more preferably a phospholipid, even more preferably phosphoacylglycerol such as hydrogenated lecithin.

Preferably the microcapsules containing releasable colorant(s) are multi-layered microcapsules containing releasable colorant(s), said microcapsules comprising:

- an uncolored core consisting in one organic material, and
- a multi-layered coating surrounding said core and comprising at least one organic inner layer and one organic outer layer of different color and entrapping respectively at least one colorant.

According to an embodiment, each layer from the microcapsule contains at least one specific colorant or a specific blend of colorant(s).

According to another embodiment, the outer layer from the microcapsule contains at least one specific colorant or a specific blend of colorant(s).

Particularly the colorants are pigments, preferably selected from the group consisting of metallic oxides.

According to an embodiment, one layer from the microcapsule contains iron oxides and titanium dioxide ($TiO_2$) as colorants.

According to an embodiment, one layer from the microcapsule only contains titanium dioxide ($TiO_2$) as colorant.

The composition may comprise at least 0.1% to 20% by weight, preferably between 0.5% and 15% by weight and in particular between 2 and 10 % by weight of microcapsules based on weight of the composition.

The composition according to the invention may further comprise from 0.1 to 70% by weight relative to the weight of the composition, of additional cosmetic ingredient(s) selected from oils particularly volatile and non-volatile silicon or hydrocarbon oils, surfactants, fillers, gelifying agents, thickening agents, film forming agents, polymers, preservatives, silicone elastomer, self-tanning agents, additional non-entrapped colorants, cosmetic actives, pH regulators, perfumes, UV filters and mixtures thereof.

The composition according to the invention, which is preferably a makeup foundation, provides a strong moisturizing and freshness sensation, creamy texture with very comfortable feeling during application, and sheer natural makeup result after application. At the end, all these features help to deliver a very good balance of skincare efficacy perception (creamy, moisturization and freshness) as well as makeup efficacy (proper coverage and natural radiance). Moreover the composition according to the invention may present a sunscreen effect.

Advantageously the microcapsules are deformable in the presence of the aqueous phase.

Advantageously the microcapsules inside the composition are breakable under pressure at the application on the keratinic materials.

The present invention is also directed to a cosmetic process for caring for and/or making up keratinic materials, comprising application on said keratinic materials in particular on the skin of a composition according to the invention.

The term "physiologically acceptable medium" is intended to denote a medium that is particularly suitable for applying a product of the invention to keratin materials, especially the skin and more particularly facial skin.

The word "capsule" is also used to mention "microcapsule".

The "physiologically acceptable medium" comprises the aqueous phase used according to the present invention.

For the purposes of the present invention, the term "keratin material" is intended to cover the skin, mucous membranes such as the lips, the nails and the eyelashes. The skin and the lips, in particular facial skin, are most particularly considered according to the invention.

As emerges from the examples that follow, compositions in accordance with the invention prove to be advantageous in several aspects.

Encapsulation of the colorants prevents undesirable re-agglomeration of pigments during manufacture and prolonged storage of the cosmetic compositions.

Without wishing to be bound to any theory, it appears that in the composition according to the invention, encapsulated colorants adsorb less water than in conventional composition, so that the freshness and moisturizing sensation as well as the good applicability of the composition remains maintained.

As the microcapsules of the invention have the ability of swelling or softening in contact of an aqueous phase as defined hereunder, they are advantageously deformable when applied on a keratin material and consequently provide a soft feeling to the user. Furthermore, their low size contributes to not create any discomfort or unfavourable, grainy, feeling when applied.

However, the microcapsules of the invention are soft enough to rupture upon very slight rubbing or pressing

on the skin in order to release their content but, nevertheless, are durable enough to avoid destruction of the coating during manufacture, even during an industrial process, and storage of corresponding change-color composition.

In addition, the microcapsule of the invention allows the use of regular equipment for the preparation of the compositions of the invention because no coloring of the apparatus occurs during the manufacturing process.

Accordingly, the microcapsules of the present invention are particularly interesting since they mask the original color of the encapsulated colorants, increase the stability of these colorants against degradation, and prevent undesirable release of the encapsulated colorants into the composition during the manufacturing process and prolonged storage.

At last, the compositions of the present invention also have the advantage of conferring a good playtime that is they are easy to apply and lead to the expected result in terms of cosmeticity.

According to another of its aspects, a subject of the present invention is also directed to a cosmetic process comprising at least the steps consisting in applying at least part of a composition according to the invention on the surface of a keratin material, in particular the skin.

According to the invention, the "color changing composition" means a composition wherein the color before application is different from the color after application, this difference being visible to the naked eyes.

In particular, this color changing composition may be linked to a color-difference ΔE in CIE Lab system 1976 (ΔE before/after application) value.

The ΔE is defined by the equation:

$$\Delta E^* = \sqrt{((L_1 - L_2)^2 + (a_1 - a_2)^2 + (b_1 - b_2)^2}$$

:

wherein $L_1,a_1,b_1$ are the parameters in the colorimetric space of the 1st color (composition before application) and $L_2,a_2,b_2$ the ones for the 2nd color (composition after the application and homogenization on the keratinic material).

These values may be measured by spectrophotometer or with a Chrosmasphere (for composition applied on skin).

The color changing composition according to the invention may be characterized as having a ΔE before/after application superior to 1, in particular superior or equal to 2, preferably superior or equal to 3.

## MICROCAPSULES CONTAINING RELEASABLE COLORANT(S)

The term "microcapsule", as used herein, refers to a spherical microcapsule containing at least one layered coating entrapping at least one colorant and surrounding a core chemically different from the coating. Microcapsules are distinct from microspheres, which consist of spherical homogeneous matrix.

According to an embodiment, the "at least one layered coating" is a multi-layered coating preferably an organic multi-layered coating.

The term "multi-layer microcapsule" refers to a microcapsule consisting of a core surrounded by a coating based on one or more inner layer(s) and one outer layer. The one or more inner layer(s) forming the multi-layer coating of the multi-layer microcapsule and the single outer layer of the microcapsule may be formed of the same or different wall-forming organic compound(s).

The microcapsule according to the invention comprises a core also called "inner core" surrounded by a coating based on one or more layer(s). In a preferred embodiment, the microcapsule is a 'multi-layers' microcapsule, comprising at least one inner layer and one outer layer. The one or more inner layer(s) forming the multi-layer coating of the multi-layer microcapsule and the single outer layer of the microcapsule may be formed of the same or different wall-forming organic compound(s).

In a particular embodiment the inner layer and the outer layer are formed of the same wall forming organic compounds, the core is then surrounded by a one layer coating.

In one embodiment, the outer layer does not comprise any colorant. In another embodiment, the outer layer comprises at least one colorant.

The term "wall-forming organic compound" refers to an organic compound or a combination of two or more different organic compounds as defined herein, which form a component of the layer(s) of the microcapsules. In a preferred embodiment, the 'wall-forming organic compound' comprises at least one polymer.

The term "colorant" refers to organic pigments such as synthetic or natural dyes selected from any of the well known FD&C or D&C dyes, inorganic pigments such as metal oxides, or lakes and any combination (blend) thereof. Accordingly, the colorant useful according to the present invention may be oil-soluble or oil-dispersible or with limited solubility in water.

In preferred embodiments, the colorant is an inorganic pigment, more preferably a metal oxide.

In particular, the average particle size may be from 50 to 1000 Mesh (around 400μm to 10μm), in particular from 60 to 200 Mesh (around 250μm to 75μm) as measured by the sieving test method or observed by microscope.

Preferably, a composition according to the invention may comprise from 0.1% to 20% by weight and preferably from 0.5% to 15% by weight of microcapsules relative to the total weight of the said composition.

In particular for a skin care composition according to the invention, the amount of microcapsules will range from 0.1% to 5%, preferably from 0.2% to 3% by weight relative to the total weight of composition.

In particular for a make-up composition according to the invention, the amount of microcapsules will range from 0.5% to 20%, preferably from 1% to 15%, more preferably from 2% to 10% by weight relative to the total weight of composition.

According to a particular embodiment, the encapsulated colorant(s) may be present in a composition according to the invention in an amount in active matter of encapsulated pigments ranging from 0.5% to 20% by weight, in particular from 1% to 15% by weight, and more particularly from 2% to 10% by weight, of the total weight of said composition.

The microcapsules will be integrated in the cosmetic formula generally at the latest stages of the formulation and after filtering stages if any, to avoid the microcapsules being broken. Preferably, the microcapsules according to the inventions are added and mixed uniformly at temperatures under 50°C. They are mixed gently with a paddle rather than a homogenizer.

The microcapsules may be produced by several methods known to the man skilled in the art within the coating or encapsulation domain, including pelletization, granulation, coating, etc. For example, the microcapsules may be obtained by a method comprising mixture of the compounds (actives, pigments, polymers, solvents) and drying to form capsules as disclosed in WO01/35933 and WO2011/027960, or a method comprising granulation and coating by spray drying as disclosed in FR2841155, or by fluidized bed technology, which has been used in the food and pharmaceutical industry for a long time for coating and encapsulating ingredients. As an example may be cited WO2008/139053, which concerns the preparation of spheroid multilayer capsules comprising a core of sugar and concentric layers of pharmaceutical actives. Fixation of pharmaceutical actives on the core is achieved by impregnation, pulverization or projection, and then the 1$^{st}$ layer is dried before application of a second one.

Fluid bed process is disclosed for example in Teunou et al. (Fluid-Bed Coating, Poncelet, 2005, D. Food Science and Technology (Boca Raton, FL, United States), Volume 146 Issue Encapsulated and Powdered Foods, Pages 197-212). A specific feature of the fluid bed process is that it leads to coated particles wherein the core is well encapsulated, compared to spray drying, which leads to a matrix with the core material randomly dispersed in a polymer.

In a preferred embodiment, the microcapsules are obtained by fluid bed process.

According to this embodiment, preferably at least one layer of the microcapsules is obtained by fluid bed process.

In a particular embodiment, the outer layer is obtained by fluid bed process.

In another particular embodiment at least one inner layer is obtained by fluid bed process.

Most preferably, all layers are obtained by fluid bed process.

A man skilled in the art knows how to adjust air quantity, liquid quantity and temperature allowing to reproduce a capsule according to the invention.

Preferably a fluid bed process implemented according to the invention includes Würster process and/or tangential spray process. Such a process allows, contrary to a pelletization process, to prepare spherical capsules with a core surrounded by one or more circumferential layers.

When the whole process for preparing the layers surrounding the core of the microcapsules according to the invention is carried out by fluid bed process, the microcapsule layers are advantageously regular, concentric and present a homogenous thickness.

Different examples of preparation of capsules according to the invention will be given later in this description.

## I a) Core

The core is made of at least an organic material. The size of said core preferably ranges from 500 nm to 150μm in diameter.

Preferably the core is in a solid and/or crystal form at room temperature.

In a particular embodiment, the organic material is selected from organic materials having high water dissolvability. Preferably, the core is water-soluble or water-dispersible.

In a particular embodiment, the core is uncolored, *i.e.* it does not contain colorant material.

In a particular embodiment, the core is based on only one compound. This compound is organic and more preferably is a natural compound.

According to a preferred embodiment, the core is sugar-alcohol, preferably a monosaccharide-polyol advantageously selected from mannitol, erythritol, xylitol, sorbitol.

In a particular embodiment, the core is made of mannitol and more preferably exclusively made of mannitol.

According to an alternative embodiment, the core contains at least mannitol and at least one additional ingredient

being preferably a polymer selected from hydrophilic polymers. In particular, such a core may comprise mannitol and hydrophilic polymers chosen among cellulose polymers, starch polymers and their mixture, preferably their mixture.

In a preferred embodiment, the cellulose polymer is a carboxymethylcellulose and the starch polymer is a non-modified natural starch, for example corn starch.

The core may be constituted by a seed (or crystal) of one of the previous materials.

The core is preferably contained in an amount of from 1% to 50% by weight, preferably 4 to 40% by weight, in particular 5 to 30% by weight, and in particular from 10 to 20% by weight with respect to the total weight of the micro capsule.

The mannitol is preferably contained in an amount of from 2% to 100% by weight, preferably 5 to 100% by weight, and in particular 100% by weight with respect to the total weight of the core.

The mannitol is preferably contained in an amount of from 1% to 50% by weight, preferably 4% to 40% by weight, in particular 5% to 30% by weight, and in particular from 10% to 20% by weight with respect to the total weight of the microcapsule.

**I b) External Layer(s) or coating**

As disclosed previously, the core is advantageously surrounded with a coating, or external layer(s) preferably comprising at least one inner layer and one outer layer. In this latter case, these layers preferably extend concentrically in respect with the core.

The layer(s) is/are preferably organic, i.e. contain(s) at least one organic compound as wall-forming material. Preferably, the inner and/or outer layer(s) include(s) at least one polymer, and in particular a hydrophilic polymer.

***Polymer(s)***

Preferably, the microcapsule according to the invention, and in particular the external layer(s) comprise(s) hydrophilic polymers selected from the group consisting of polysaccharides and derivatives, acrylic or methacrylic acid homopolymers or copolymers or salts and esters thereof, and their mixture.

In a preferred embodiment, the microcapsule according to the invention, and in particular the external layer(s) comprise(s) hydrophilic polymers selected from the group consisting of polysaccharides and derivatives, and in particular starch polymers.

The said polymer(s) is (are) advantageously selected from (poly)(alkyl)(meth)acrylic acid and derivatives, notably (poly)(alkyl)(meth)acrylate and derivatives, preferably from alkylacrylic /alkylmethacrylic acid copolymers and their derivatives, and most preferably is a copolymer of ethyl acrylate, methyl methacrylate and low content of methacrylic acid ester with quaternary ammonium groups provided under the tradename of EUDRAGIT RSPO from Evonik Degussa.

Said polysaccharides and derivatives are preferably selected from chitosan polymers, chitin polymers, cellulose polymers, starch polymers, galactomannans, alginates, carrageenans, mucopolysaccharides, and their derivatives, and the mixture thereof.

In a preferred embodiment, the external layer(s) is/are devoid of microcrystalline cellulose.

According to one particularly preferred embodiment, said polysaccharides and their derivatives are preferably selected from the ones including one type of ose or several type of ose(s), preferably several types of oses, in particular at least D-Glucose unit(s) as ose(s), preferably starch polymers, cellulose polymers, and derivatives, and the mixture thereof.

According to a preferred embodiment, the microcapsule contains at least one hydrophilic polymer selected from the group consisting of starch and its derivatives, in particular corn starch, cellulose and its derivatives, homo- and/or co-polymer of methacrylic acid and/or methacrylic acid ester or co-polymer of (alkyl)acrylic acid and/or (alkyl)methacrylic acid and their derivatives, preferably their salts and their ester, and in particular the capsule contains polymethyl methacrylate.

According to a preferred embodiment, the microcapsule contains at least one hydrophilic polymer selected from the group consisting of starch and its derivatives, in particular corn starch

Starch usable according to the present invention is usually issued from vegetable raw materials, such as rice, soybeans, potatoes, or corn. Starch can be unmodified or (by analogy with cellulose) modified starch. In a preferred embodiment, the starch is unmodified.

Preferred homo- and /or co-polymer of methacrylic acid and/or methacrylic acid ester are those wherein the copolymer of methyl methacrylate and ethyl acrylate has a molecular weight from 750 to 850 kDa.

Cellulose derivatives include, for example, alkali celluloses carboxymethyl cellulose (CMC), cellulose esters and ethers, and aminocelluloses. In a particular embodiment, the cellulose is a carboxymethyl cellulose (CMC).

According to a preferred embodiment, the capsule contains at least starch derivative, in particular corn starch, polymethyl methacrylate, co-polymer of (alkyl)acrylic acid and/or (alkyl)methacrylic acid and their derivatives preferably their salts and their ester, and/or cellulose derivative.

Preferably, the microcapsule contains polymer(s) which are not cross-linked.

The polymer(s) may be in one or several layer(s).

In another embodiment, the polymer(s) may be in the core.

The microcapsule may contain polymer(s) in the core and/or in the layer(s).

In a particular embodiment, the polymer(s) is (are) in the core and in the layer(s).

In an embodiment, the core contains at least starch and/or cellulose derivative as polymer(s). When the starch is contained within the core, it represents the main ingredient of such a core, i.e. the weight amount of starch is greater than the respective amount of other compounds of the core.

The polymer may represent from 0.5 to 20% by weight of the microcapsule, in particular from 1 to 10% by weight, preferably from 2 to 8% by weight of the microcapsule.

The different layers forming the coating may be based on identical or different polymers. Advantageously, they will be formed from the same polymer.

In contrast, the layers will be advantageously differently colored.

This different color may be obtained through the use of different colorants but also the use of different concentrations in at least one colorant when the colorant will be the same for two layers.

In a particular embodiment, the outer layer contains at least one colorant.

In another embodiment, the outer layer does not contain any colorant.

As previously stated, "colorant" includes any organic or inorganic pigment or colorant approved for use in cosmetics by CTFA and the FDA used in cosmetic formulations.

Thus the term "colorant" refers to organic pigments such as synthetic or natural dyes selected from any of the well known FD&C or D&C dyes, to inorganic pigments such as metal oxides, or lakes such as the ones based on cochineal carmine, barium, strontium, calcium or aluminum and any combination (blend) thereof. Such colorants are detailed here-after.

In a particular embodiment, the colorant may be water-soluble or water-dispersible.

In another embodiment, the colorant useful according to the present invention may be oil-soluble or oil-dispersible or with limited solubility in water.

In preferred embodiments, the colorant is an inorganic pigment, more preferably a metal oxide.

Advantageously, the colorants of the multi-layer microcapsules are primary metal oxides selected from iron oxides, titanium dioxide, aluminum oxide, zirconium oxides, cobalt oxides, cerium oxides, nickel oxides, tin oxide or zinc oxide, or composite oxides, more preferably an iron oxide selected from red iron oxide, yellow iron oxide or black iron oxide, or a mixture thereof.

The layer(s) may also contain lakes corresponding to an organic colorant secured to a substrate. Such (a) lake(s) is (are) advantageously chosen among the here-below material, and their mixture(s):

- carmin of cochineal;
- organic pigments of azoic, anthraquinonic, indigoid, xanthenic, pyrenic, quinolinic, triphenylmethane, fluoran colorants; Among the organic pigments may be cited those known under the following trademark references : D&C Blue n° 4, D&C Brown n° 1, D&C Green n° 5, D&C Green n° 6, D&C Orange n° 4, D&C Orange n° 5, D&C Orange n°10, D&C Orange n° 11, D&C Red n° 6, D&C Red n° 7, D&C Red n° 17, D&C Red n°21, D&C Red n° 22, D&C Red n° 27, D&C Red n° 28, D&C Red n° 30, D&C Red n°31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6 ;
- the water-insoluble salts of sodium, potassium, calcium, baryum, aluminum, zirconium, strontium, titanium, of acid colorants such as azoic, anthraquinonic, indigoids, xanthenic, pyrenic, quinolinic, triphenylmethane, fluoran colorants, these colorants may include at least one carboxylic or sulfonic acid group.

The organic lakes may also be protected by an organic support such as rosin or aluminum benzoate.

Among the organic lakes, we may in particular cite those known under the following names : D&C Red n° 2 Aluminum lake, D&C Red n° 3 Aluminum lake, D&C Red n° 4 Aluminum lake, D&C Red n° 6 Aluminum lake, D&C Red n° 6 Barium lake, D&C Red n° 6 Barium/Strontium lake, D&C Red n° 6 Strontium lake, D&C Red n° 6 Potassium lake, D&C Red n° 6 Sodium lake, D&C Red n° 7 Aluminum lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminum lake, D&C Red n° 9 Barium lake, D&C Red n° 9 Barium/Strontium lake, D&C Red n° 9 Zirconium lake, D&C Red n° 10 Sodium lake, D&C Red n° 19 Aluminum lake, D&C Red n° 19 Barium lake, D&C Red n° 19 Zirconium lake, D&C Red n° 21 Aluminum lake, D&C Red n° 21 Zirconium lake, D&C Red n° 22 Aluminum lake, D&C Red n° 27 Aluminum lake, D&C Red n° 27 Aluminum/Titanium/Zirconium lake, D&C Red n° 27 Barium lake, D&C Red n° 27 Calcium lake, D&C Red n° 27 Zirconium lake, D&C Red n° 28 Aluminum lake, D&C Red n° 28 Sodium lake D&C Red n° 30 lake, D&C Red n° 31 Calcium lake, D&C Red n° 33 Aluminum lake, D&C Red n° 34 Calcium lake, D&C Red n° 36 lake, D&C Red

n° 40 Aluminum lake, D&C Blue n° 1 Aluminum lake, D&C Green n° 3 Aluminum lake, D&C Orange n° 4 Aluminum lake, D&C Orange n° 5 Aluminum lake, D&C Orange n° 5 Zirconium lake, D&C Orange n° 10 Aluminum lake, D&C Orange n° 17 Barium lake, D&C Yellow n° 5 Aluminum lake, D&C Yellow n° 5 Zirconium lake, D&C Yellow n° 6 Aluminum lake, D&C Yellow n° 7 Zirconium lake, D&C Yellow n° 10 Aluminum lake, FD&C Blue n° 1 Aluminum lake, FD&C Red n° 4 Aluminum lake, FD&C Red n° 40 Aluminum lake, FD&C Yellow n° 5 Aluminum lake, FD&C Yellow n° 6 Aluminum lake.

The chemistry material corresponding to each of these organic colorants previously cited are mentioned in the book called «International Cosmetic Ingredient Dictionnary and Handbook », Edition 1997, pages 371 to 386 and 524 to 528, published by « The Cosmetic, Toiletry, and Fragrance Association », of which the content is hereby incorporated by reference in the present specification.

According to a preferred embodiment, the lake(s) is/are selected from carmin of cochineal and the water-insoluble salts of sodium, potassium, calcium, barium, aluminum, zirconium, strontium, titanium, of acid colorants such as azoic, anthraquinonic, indigoid, xanthenic, pyrenic, quinolinic, triphenylmethane, fluoran colorants, being given that these colorants may include at least one carboxylic or sulfonic acid group, and their mixture.

According to a preferred embodiment, the lake(s) is/are selected from carmin of cochineal and the water-insoluble salts of sodium, calcium, aluminum, and their mixture.

As lake incorporating carmine we may cite the commercial references : CARMIN COVALAC W 3508, CLOISONNE RED 424C et CHROMA-LITE MAGENTA CL4505.

The water-insoluble aluminum salts are preferably selected from FDC Yellow N°5 aluminum lake, le FDC Blue N°1 aluminum lake, le FDC Red N°40 aluminum lake, le FDC Red N°30 aluminum lake, le FDC Green N°5 aluminum lake, and their mixtures. As compound incorporating such inorganic lake may notably be cited the commercial references : INTENZA FIREFLY C91-1211, INTENZA AZURE ALLURE C91-1251, INTENZA THINK PINK C91-1236

The water-insoluble calcium salts are preferably selected from Red N°7 calcium lake. As compound incorporating such inorganic lake may notably be cited the commercial references: INTENZA MAGENTITUDE C91-1234, INTENZA HAUTE PINK C91-1232, INTENZA RAZZLED ROSE C91-1231, INTENZA AMETHYST FORCE C91-7231, INTENZA PLUSH PLUM C91-7441, INTENZA ELECTRIC CORAL C91-1233, FLORASOMES-JOJOBA-SMS-10% CELLINI RED-NATURAL and their mixture.

The water-insoluble sodium salts are preferably selected from Red N°6 sodium lake and Red N°28 sodium lake, and their mixture. E As compound incorporating such inorganic lake may notably be cited the commercial references: INTENZA MANGO TANGO C91-1221 and INTENZA NITRO PINK C91-1235.

In preferred embodiments, the colorant is an inorganic colorant.

In a preferred embodiment, the colorant is a metallic oxide. Such metallic oxide is preferably selected from iron oxides, titanium oxides, and mixtures thereof.

The color-changing compositions of the invention may comprise a mixture of two or more colorants in the microcapsules, either encapsulated individually and/or one or more blends of colorants encapsulated within the multilayer microcapsules.

In accordance with this specific embodiment, each layer of the microcapsule may contain at least one specific colorant or a specific blend of colorant(s).

In accordance with this specific embodiment, the color-changing composition of the invention comprises two or more microcapsules of the invention having different colors.

A person skilled in the art knows how to choose colorants and combinations of colorants to produce a desired color effect or color change.

As stated previously, the microcapsules of the invention contain preferably at least titanium dioxide and/or iron oxides in their coating, preferably at least titanium dioxide.

In a preferred embodiment, the microcapsules of the invention contain preferably at least titanium dioxide and iron oxides in their coating.

According to a specific embodiment, the outer layer of said microcapsules contains titanium dioxide and more preferably as only colorant.

In one of its aspects, the color-changing composition of the present invention is an opalescent composition, the composition is colored and contains white or colored microcapsules.

According to a specific embodiment, the outer layer of said microcapsules contains organic pigments or iron oxides.

The colorants in the microcapsules are present in amounts ranging from 20% to 90% by weight, preferably from 30% to 80% by weight, more preferably from 50% to 75% by weight relative to the total weight of the microcapsule.

In a particular embodiment, the microcapsules contain metallic oxides selected from iron oxides, titanium oxides, and mixtures thereof, present in an amount ranging from 20% to 90% by weight, preferably from 30% to 85% by weight, more preferably from 50% to 85% by weight relative to the total weight of the microcapsule.

In particular the titanium oxide may be present from 28% to 80% by weight, preferably from 30% to 75% by weight, and more preferably from 30 to 50% by weight, relative to the total weight of the microcapsule.

In particular the iron oxides may be present from 5% to 75% by weight, preferably 8% to 65% by weight relative to the total weight of the microcapsule. In a particular embodiment, the iron oxides are present in an amount higher than 15% by weight, preferably higher than 30% by weight, and in particular from 40% to 65% by weight relative to the total weight of the microcapsule.

In a preferred embodiment, in at least one layer, and preferably in every layer, the colorants are the main ingredients, *i.e.* represent at least 40% by weight of the layer(s), preferably at least 75% by weight of the layer(s), more preferably at least 95% by weight of the layer(s).

In a preferred embodiment the mean thickness of the titanium dioxide layer ranges from 5μm to 150μm.

***Livid-based material***

The inner and/or outer layer(s) may also include advantageously at least one lipid-based material.

According to a particular embodiment of this invention, such a lipid-based material may have amphiphilic properties, that is to say having an apolar part and a polar part.

Such lipid-based material can include at least one or several $C_{12}$-$C_{22}$ fatty acid chain(s) such as those selected from stearic acid, palmitic acid, oleic acid, linoleic acid, linolenic acid, etc., and mixtures thereof. Preferably these fatty acids chains are hydrogenated. Eventually, these fatty acid chains may be the apolar part of a lipid-based material.

Such lipid-based material is preferably selected from phospholipids. These phospholipids are preferably selected from phosphoacylglycerol, more preferably selected from lecithins, and are in particular hydrogenated lecithin.

The lipid based material may represent from 0.05 to 5% by weight of the microcapsule, in particular from 0.1 to 1% by weight of microcapsule.

By combining three or more compounds (ex: sugar alcohols, polymers, lipid-based material) in the microcapsule of different hardness and/or water solubility, it is possible to adjust the time required for colorant-encapsulated microcapsules to break down on the skin so that, by varying the method or intensity of application onto the skin, it is possible to adjust the preferred coloration or gradation pattern.

Thus, according to a preferred embodiment, the multi-layer coating contains at least starch as polymer and at least one lipid-based material, which is preferably lecithin.

According to an advantageous embodiment the microcapsules according to the invention include at least one monosaccharide or its derivative and at least one polysaccharide or its derivatives.

According to a preferred embodiment, the microcapsules include a core comprising a monosaccharide derivative and a coating comprising a polysaccharide (or its derivative) including one type of ose or several type of ose(s), preferably several types of oses.

According to a more preferably embodiment, the microcapsules include a core comprising a monosaccharide polyol, preferably selected from mannitol, erythritol, xylitol, sorbitol, and a coating comprising a polysaccharide (or its derivative) including as ose(s) at least one or more D-Glucose unit(s).

According to a preferred embodiment, the microcapsules include three or more colorants in different layers.

According to a preferred embodiment, the microcapsules additionally include a lipid-based material chosen from phospholipids, advantageously selected from phosphoacylglycerol and in particular from lecithins.

In a particular embodiment, the microcapsule contains mannitol, starch polymer and a lipid-based material.

Referring to figure 1, according to a preferred embodiment, the present invention advantageously provides a color-changing composition comprising microcapsules having a size ranging from 10 μm to 800 μm, preferably from 50 μm to 600 μm and more preferably from 60μm to 250μm in diameter of the microcapsule, comprising:

a) a core (A), preferably having a size ranging from 500 nm to 150 μm in diameter, which preferably does not contain any colorant, and comprising at least one organic core preferably selected from at least one sugar alcohol preferably a monosaccharide-polyol advantageously selected from mannitol, erythritol, xylitol, sorbitol, and mixture thereof;
b) one first layer (B) surrounding said core comprising:

  i. at least one colorant, preferably iron oxide(s), and
  ii. a binder selected from at least one polymer, at least one lipid-based material, and their mixture, preferably their mixture ;

c) one second layer (C) surrounding said first layer (B), preferably having a thickness of 5 to 500μm, comprising:

  i. titanium dioxide particles, and
  ii. a binder selected from at least one polymer, at least one lipid-based material, and their mixture, preferably their mixture ;

d) optionally one third layer (D) surrounding said second layer (C) comprising:

i. at least one colorant, and
ii. a binder selected from at least one polymer, at least one lipid-based material, and their mixture, preferably their mixture ;

e) optionally one fourth layer (E) surrounding said third layer (D), if any, or surrounding said second layer (C) comprising

- at least one wall-forming polymer preferably selected from polysaccharides such as cellulose derivatives, in particular cellulose ether and cellulose ester, from (poly)(alkyl)(meth)acrylic acid and derivatives, notably (poly)(alkyl)(meth)acrylate and derivatives, and preferably from alkylacrylic /alkylmethacrylic acid copolymers and their derivatives.

As examples of commercially available microcapsules to be used in the composition of the invention, we may refer to the following microcapsules produced by Korea Particle Technology KPT under the commercial name :
Magic50-BW0105 from KPT: ash gray spherical microcapsule containing mannitol, iron oxide red, iron oxide yellow, iron oxide black, hydrogenated lecithin, titanium dioxide, zea mays (corn) starch, having 60-200 Mesh particle size.
The microcapsules suitable for the present invention are stable into the compositions according to the present invention, preferably at high temperatures, for instance greater than or equal to 40°C, for example for one month, better two months and still better three months in an oven at 45°C or for 15 days in an oven at 60°C.
In a preferred embodiment, the microcapsules according to the present invention present an appropriate softening kinetics.
That is preferably, at least three hours after being in contact with the other compounds of the formula, the hardness of the microcapsules is advantageously from 5 to 50 grams, more preferably from 6 to 20 grams and still more preferably from 7 to 10 grams. Such hardness is in conformity with an industrial process for preparing the cosmetic compositions including such microcapsules.
Such values of softening kinetics and hardness allow to provide not only aesthetic microcapsules but also overall aesthetic compositions.
Particularly, the composition may lead to different shades or color gradations depending on the intensity of the rubbing. The compositions may advantageously present a high chromaticity C* as measured in the in CIE Lab system 1976.

## PIGMENTS

The composition of the present invention comprises at least one pigment chosen from organic pigments and non-lamellar mineral pigments.
Said pigment chosen from organic pigments and non-lamellar mineral pigments is not encapsulated in the microcapsules.
The pigment(s) and the microcapsules are present in the composition in a weight ratio of pigment / microcapsules > 0,5, preferably in a weight ratio of pigment / microcapsules > 1 and most preferably in a weight ratio of pigment / microcapsules > 1,5.
This pigment is, like the colorant contained in the microcapsules, a pigment approved for use in cosmetics by CTFA and the FDA used in cosmetic formulations.
In a particular embodiment, said pigment may be water-soluble or water-dispersible.
In another embodiment, the pigment may be oil-soluble or oil-dispersible or with limited solubility in water.
In preferred embodiments, this pigment is a non-lamellar mineral pigment, more preferably a metal oxide, such as iron oxides, titanium dioxide, aluminum oxide, zirconium oxides, cobalt oxides, cerium oxides, nickel oxides, tin oxide, chromium oxide or zinc oxide, or composite oxides, particularly an iron oxide selected from red iron oxide, yellow iron oxide or black iron oxide, or a mixture thereof, or even Prussian blue, manganese violet, ultramarine blue or chromium hydrate.
According to a specific embodiment, the pigment used according to the invention is an organic pigment chosen from synthetic or natural dyes particularly selected from any of the well known FD&C or D&C dyes. The organic pigment may also be a diketopyrrolopyrroles (DPP) as the ones described in EP-A-542669, EP-A-787730, EP-A-787731 and WO-A- 96/08537.
The pigment may also be a lake corresponding to an organic colorant secured to a substrate. Such (a) lake(s) is (are) advantageously chosen among the here-below material, and their mixture(s) :

- carmin of cochineal;
- organic pigments of azoic, anthraquinonic, indigoid, xanthenic, pyrenic, quinolinic, triphenylmethane, fluoran colorants; Among the organic pigments may be cited those known under the following trademark references : D&C Blue n° 4, D&C Brown n° 1, D&C Green n° 5, D&C Green n° 6, D&C Orange n° 4, D&C Orange n° 5, D&C Orange n°10, D&C Orange n° 11, D&C Red n° 6, D&C Red n° 7, D&C Red n° 17, D&C Red n°21, D&C Red n° 22, D&C Red n° 27, D&C Red n° 28, D&C Red n° 30, D&C Red n°31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6 ;
- the water-insoluble salts of sodium, potassium, calcium, baryum, aluminum, zirconium, strontium, titanium, of acid colorants such as azoic, anthraquinonic, indigoids, xanthenic, pyrenic, quinolinic, triphenylmethane, fluoran colorants, these colorants may include at least one carboxylic or sulfonic acid group.

The organic lakes may also be protected by an organic support such as rosin or aluminum benzoate.

Among the organic lakes, we may in particular cite those known under the following names : D&C Red n° 2 Aluminum lake, D&C Red n° 3 Aluminum lake, D&C Red n° 4 Aluminum lake, D&C Red n° 6 Aluminum lake, D&C Red n° 6 Barium lake, D&C Red n° 6 Barium/Strontium lake, D&C Red n° 6 Strontium lake, D&C Red n° 6 Potassium lake, D&C Red n° 6 Sodium lake, D&C Red n° 7 Aluminum lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminum lake, D&C Red n° 9 Barium lake, D&C Red n° 9 Barium/Strontium lake, D&C Red n° 9 Zirconium lake, D&C Red n° 10 Sodium lake, D&C Red n° 19 Aluminum lake, D&C Red n° 19 Barium lake, D&C Red n° 19 Zirconium lake, D&C Red n° 21 Aluminum lake, D&C Red n° 21 Zirconium lake, D&C Red n° 22 Aluminum lake, D&C Red n° 27 Aluminum lake, D&C Red n° 27 Aluminum/Titanium/Zirconium lake, D&C Red n° 27 Barium lake, D&C Red n° 27 Calcium lake, D&C Red n° 27 Zirconium lake, D&C Red n° 28 Aluminum lake, D&C Red n° 28 Sodium lake D&C Red n° 30 lake, D&C Red n° 31 Calcium lake, D&C Red n° 33 Aluminum lake, D&C Red n° 34 Calcium lake, D&C Red n° 36 lake, D&C Red n° 40 Aluminum lake, D&C Blue n° 1 Aluminum lake, D&C Green n° 3 Aluminum lake, D&C Orange n° 4 Aluminum lake, D&C Orange n° 5 Aluminum lake, D&C Orange n° 5 Zirconium lake, D&C Orange n° 10 Aluminum lake, D&C Orange n° 17 Barium lake, D&C Yellow n° 5 Aluminum lake, D&C Yellow n° 5 Zirconium lake, D&C Yellow n° 6 Aluminum lake, D&C Yellow n° 7 Zirconium lake, D&C Yellow n° 10 Aluminum lake, FD&C Blue n° 1 Aluminum lake, FD&C Red n° 4 Aluminum lake, FD&C Red n° 40 Aluminum lake, FD&C Yellow n° 5 Aluminum lake, FD&C Yellow n° 6 Aluminum lake.

According to a preferred embodiment, the lake(s) is/are selected from carmin of cochineal and the water-insoluble salts of sodium, potassium, calcium, barium, aluminum, zirconium, strontium, titanium, of acid colorants such as azoic, anthraquinonic, indigoid, xanthenic, pyrenic, quinolinic, triphenylmethane, fluoran colorants, being given that these colorants may include at least one carboxylic or sulfonic acid group, and their mixture.

According to a preferred embodiment, the lake(s) is/are selected from carmin of cochineal and the water-insoluble salts of sodium, calcium, aluminum, and their mixture.

As lake incorporating carmine we may cite the commercial references : CARMIN COVALAC W 3508, CLOISONNE RED 424C et CHROMA-LITE MAGENTA CL4505.

The water-insoluble aluminum salts are preferably selected from FDC Yellow N°5 aluminum lake, le FDC Blue N°1 aluminum lake, le FDC Red N°40 aluminum lake, le FDC Red N°30 aluminum lake, le FDC Green N°5 aluminum lake, and their mixtures. As compound incorporating such inorganic lake may notably be cited the commercial references : INTENZA FIREFLY C91-1211, INTENZA AZURE ALLURE C91-1251, INTENZA THINK PINK C91-1236

The water-insoluble calcium salts are preferably selected from Red N°7 calcium lake. As compound incorporating such inorganic lake may notably be cited the commercial references: INTENZA MAGENTITUDE C91-1234, INTENZA HAUTE PINK C91-1232, INTENZA RAZZLED ROSE C91-1231, INTENZA AMETHYST FORCE C91-7231, INTENZA PLUSH PLUM C91-7441, INTENZA ELECTRIC CORAL C91-1233, FLORASOMES-JOJOBA-SMS-10% CELLINI RED-NATURAL and their mixture.

The water-insoluble sodium salts are preferably selected from Red N°6 sodium lake and Red N°28 sodium lake, and their mixture. E As compound incorporating such inorganic lake may notably be cited the commercial references: INTENZA MANGO TANGO C91-1221 and INTENZA NITRO PINK C91-1235.

The amount of non-encapsulated pigment(s) in the composition of the invention may range, for example, in terms of active material, from 1% to 20% by weight and preferably from 2% to 15%, more preferably from 4% to 12%, and advantageously from 5% to 10% by weight, relative to the total weight of the composition.

## GELIFYING AGENT

In a preferred embodiment, the composition of the present invention comprises a gelifying agent, preferably chosen from polymers comprising 2-acrylamido-2-methylpropanesulphonic acid units and heterogeneous polysaccharides.

The heterogeneous polysaccharide can be a heterogeneous polysaccharide comprising at least one fucose unit, comprising in particular fucose, galactose and galacturonic acid units, in particular a linear sequence of α-L-fucose, of α-D-galactose and of galacturonic acid. Such a polysaccharide is available in particular in the form of a 1% solution in water from Solabia under the trade name Fucogel 1000 PP®.

The amount of heterogeneous polysaccharide(s) in the composition of the invention may range, for example, in terms of active material, from 0.01% to 15% by weight and preferably from 0.1% to 5% by weight, relative to the total weight of the composition.

More preferably it is a 2-acrylamido-2-methylpropanesulphonic acid polymer.

In the present application, the expression "polymer comprising 2-acrylamido-2-methylpropanesulphonic acid units" (AMPS) is intended to mean both homopolymers and copolymers, and both crosslinked polymers and non-crosslinked polymers.

Preferably the at least one 2-acrylamido-2-methylpropanesulphonic acid polymer is a 2-acrylamido-2-methylpropanesulphonic acid homopolymer.

They are water-soluble or water-dispersible or water-swellable polymers.

Preferably, the AMPS polymers used in accordance with the invention may be partially or completely neutralized with an inorganic base (sodium hydroxide, potassium hydroxide, aqueous ammonia) or an organic base such as mono-, di- or triethanolamine, an aminomethylpropanediol, N-methylglucamine or basic amino acids such as arginine and lysine, and mixtures of these compounds. They are generally neutralized. In the present invention, the term "neutralized" is intended to mean polymers that have been completely or almost completely neutralized, i.e. at least 90% neutralized.

The AMPS polymers used in the composition of the invention generally have a number-average molecular weight ranging from 1000 to 20 000 000 g/mol, preferably ranging from 20 000 to 5 000 000, and even more preferably from 100 000 to 1 500 000 g/mol.

When the polymers are crosslinked, the crosslinking agents may be chosen from compounds with an olefinic polyunsaturation commonly used for crosslinking polymers obtained by radical polymerization. As crosslinking agents, mention may, for example, be made of divinylbenzene, diallyl ether, dipropylene glycol diallyl ether, polyglycol diallyl ethers, triethylene glycol divinyl ether, hydroquinone diallyl ether, ethylene glycol di(meth)acrylate, tetraethylene glycol di(meth)acrylate, trimethylolpropane triacrylate, methylenebisacrylamide, methylenebismethacrylamide, triallylamine, triallyl cyanurate, diallyl maleate, tetraallylethylenediamine, tetraallyloxyethane, trimethylolpropane diallyl ether, allyl (meth)acrylate, allyl ethers of alcohols of the sugar series, or other allyl or vinyl ethers of polyfunctional alcohols, and also allyl esters of phosphoric acid derivatives and/or vinylphosphonic acid derivatives, or mixtures of these compounds. According to a preferred embodiment of the invention, the crosslinking agent is chosen from methylenebisacrylamide, allyl methacrylate and trimethylolpropane triacrylate (TMPTA). The degree of crosslinking generally ranges from 0.01 to 10 mol%, and more particularly from 0.2 to 2 mol%, relative to the polymer.

The AMPS homopolymers preferred for use in the composition of the invention are crosslinked and neutralized, and they can be obtained according to the preparation process comprising the following steps:

(a) 2-acrylamido-2-methylpropanesulphonic acid in free form is dispersed or dissolved in a solution of tert-butanol or of water and tert-butanol;

(b) the solution or the dispersion of monomer obtained in (a) is neutralized with one or more inorganic or organic bases, preferably aqueous ammonia $NH_3$, in an amount that makes it possible to obtain a degree of neutralization of the sulphonic acid functional groups of the polymer ranging from 90% to 100%;

(c) the crosslinking monomer(s) is (are) added to the solution or dispersion obtained in (b);

(d) a conventional radical polymerization is carried out in the presence of free radical initiators at a temperature ranging from 10 to 150°C; the polymer precipitating from the tert-butanol-based solution or dispersion.

The AMPS homopolymers that are more particularly preferred comprise, distributed randomly, units of general formula (I) below:

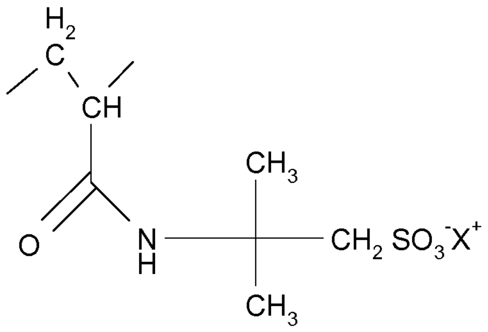

(I)

in which $X^+$ denotes a proton, an alkali metal cation, an alkaline earth metal cation or the ammonium ion, it being possible for no more than 10 mol% of the $X^+$ cations to be $H^+$ protons;
and crosslinking units originating from at least one monomer having at least two olefinic double bonds.

The homopolymers used according to the invention and that are more particularly preferred comprise from 90% to 99.9% by weight, and preferably from 98% to 99.5% by weight of units of formula (I), and from 0.01% to 10% by weight, preferably from 0.2% to 2% by weight of crosslinking units, the proportions by weight being defined relative to the total weight of the polymer.

As a preferred homopolymer of this type, mention may be made of the crosslinked and neutralized 2-acrylamido-2-methylpropanesulphonic acid homopolymer sold by the company Clariant under the trade name "Hostacerin AMPS" (INCI name: Ammonium Polyacryldimethyltauramide).

The 2-acrylamido-2-methylpropanesulphonic acid (AMPS) copolymers that can be used in the composition of the invention may be chosen in particular from:

1) crosslinked anionic copolymers of acrylamide or methacrylamide and of 2-acrylamido-2-methylpropanesulphonic acid, in particular those which are in the form of a W/O emulsion, such as those sold under the name Sepigel 305 by the company Seppic (INCI name: Polyacrylamide/C13-14 Isoparaffin/Laureth-7), or under the name Simulgel 600 by the company Seppic (INCI name: Acrylamide/Sodium acryloyldimethyltaurate copolymer/isohexadecane/Polysorbate 80),
2) copolymers of (meth)acrylic acid or of (meth)acrylate and of 2-acrylamido-2-methylpropanesulphonic acid, in particular those which are in the form of a W/O emulsion, such as those sold under the name Simulgel NS by the company Seppic (copolymer of sodium acrylamido-2-methylpropanesulphonate/hydroxylethyl acrylate in an inverse emulsion at 40% in polysorbate 60 and squalene) (INCI name: Hydroxyethyl acrylate/sodium acryloyldimethyltaurate copolymer/squalene/polysorbate 60) or those sold under the name Simulgel EG by the company Seppic (copolymer of acrylic acid/acrylamido-2-methylpropanesulphonic acid in the form of a sodium salt in an inverse emulsion at 45% in isohexadecane/water) (INCI name: sodium acrylate/sodium acryloyldimethyltaurate copolymer/isohexadecane/Polysorbate 80),
3) copolymers of 2-acrylamido-2-methylpropanesulphonic acid and of vinylpyrrolidone or of vinylformamide, such as the products sold under the names Aristoflex AVC by the company Clariant,
4) copolymers of 2-acrylamido-2-methylpropanesulphonic acid containing a hydrophobic unit, in particular the copolymers comprising a 2-acrylamido-2-methylpropanesulphonic acid unit of formula (I) as defined above, and at least one hydrophobic unit of formula (II)

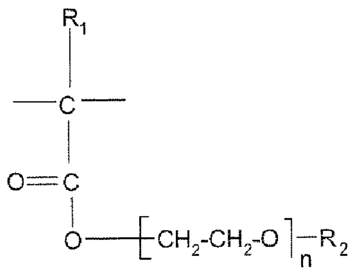

(II)

in which n denotes a number of moles which is an integer ranging from 3 to 100, preferably from 3 to 50, and more preferably from 7 to 25, $R_1$ is hydrogen or a methyl radical, and $R_2$ denotes a linear or branched alkyl radical containing from 6 to 30 carbon atoms, preferably from 10 to 22 carbon atoms, and better still from 14 to 22 carbon

atoms.

In these copolymers, the AMPS unit of formula (I) represents in general from 80 to 99 mol%, and preferably from 85 to 99 mol%, and the unit of formula (II) represents in general from 1 to 20 mol%, and preferably from 1 to 15 mol%.

As AMPS copolymers containing a hydrophobic unit, mention may in particular be made of the copolymer of AMPS and of ethoxylated $C_{12}$-$C_{14}$ alkyl methacrylate (non-crosslinked copolymer obtained from Genapol LA-070 and from AMPS) (INCI name: Ammonium Acryloyldimethyltaurate/Laureth-7 Methacrylate Copolymer) sold under the name Aristoflex LNC by the company Clariant, the copolymer of AMPS and of ethoxylated stearyl methacrylate (25 EO) (copolymer crosslinked with trimethylolpropane triacrylate, obtained from Genapol T-250 and from AMPS) (INCI name: Ammonium Acryloyldimethyltaurate/Steareth 25 Methacrylate Crosspolymer) sold under the name Aristoflex HMS by the company Clariant, and the copolymer of AMPS and of ethoxylated $C_{16}$-$C_{18}$ alkyl methacrylate (INCI name: Ammonium Acryloyldimethyltaurate/Steareth-8 Methacrylate Copolymer), a non-crosslinked copolymer obtained from Genapol T-080 and from AMPS, sold under the name Aristoflex SNC by the company Clariant.

The amount of 2-acrylamido-2-methylpropanesulphonic acid polymer(s) in the composition of the invention may range, for example, in terms of active material, from 0.01% to 15% by weight and preferably from 0.1% to 5% by weight, relative to the total weight of the composition.

In a particular embodiment, the composition of the invention is a O/W emulsion and the amount of 2-acrylamido-2-methylpropanesulphonic acid polymer(s) is, in terms of active material, from 0.2% to 5% by weight, preferably from 0.5% to 3% by weight, and more preferably from 0.5% to 2% relative to the total weight of the composition.

## AQUEOUS PHASE

Preferably, the microcapsules of the invention need to be in contact with an aqueous phase comprising water.

The aqueous phase is present in an amount of at least 50% by weight, preferably at least 60% by weight, and more preferably at least 65% by weight relative to the total weight of the composition. Advantageously, the aqueous phase may be present in a content ranging from 50% to 99 % by weight, preferably from 60% to 95% more preferably from 65% to 90% by weight relative to the total weight of the said composition.

Advantageously, water is present in an amount of at least 20% by weight, preferably at least 30% by weight, more preferably at least 40% by weight and advantageously at least 50% by weight relative to the weight of the composition.

Generally water is present in an amount ranging from 20% to 90% by weight, preferably 30% to 85% by weight and more preferably from 40 to 80% by weight, relative to the weight of the composition.

This aqueous phase is particularly advantageous for imparting and/or improving deformability to the microcapsules of the invention.

Advantageously this aqueous phase acts as a swelling agent or as a softening agent towards the microcapsules without breaking them. The microcapsules are not inert when placed in this aqueous phase either they swell : their diameter significantly increases with an optional softening of the microcapsules, or the microcapsules significantly soften without increasing of the diameter, they become more malleable and easier to break when applied onto the skin.

The aqueous phase used in the composition according to the invention is able to act on the softening kinetics of the microcapsules and more particularly it allows to obtain a good balance between softening kinetics and hardness.

As a consequence, said aqueous phase is particularly advantageous for softening the microcapsules suitable for the present invention, in an appropriate way, since said aqueous phase plays a role on softening kinetics of said microcapsules.

Advantageously this aqueous phase acts as a swelling agent or as a softening agent towards the microcapsules preferably without breaking them or without triggering colorant leakage.

The aqueous phase comprises advantageously a water-soluble solvent.

In the present invention, the term "water-soluble solvent" denotes a compound that is liquid at room temperature and water-miscible (miscibility with water of greater than 50% by weight at 25°C and atmospheric pressure).

The water-soluble solvents that may be used in the composition of the invention may also be volatile.

In one embodiment, the composition of the invention may contain an aqueous phase comprising water and at least one compound chosen among polyols, in particular glycols, $C_2$-$C_8$ monoalcohols and mixtures thereof. It also may contain $C_4$ ketones and $C_2$-$C_4$ aldehydes. The composition of the invention may comprise at least one compound chosen from polyols, glycols, C2-C8 monoalcohols, and mixtures thereof in an amount ranging from 3% to 50% by weight, preferably from 5% to 45% by weight and more preferably from 10% to 45% by weight relative to the total weight of the composition.

In a preferred embodiment, the aqueous phase suitable for the present invention comprises at least one polyol particularly a glycol.

## ***Monoalcohols or Lower alcohols***

Monoalcohol or lower alcohol that is suitable for use in the invention may be a compound of linear, branched or cyclic, saturated or unsaturated alkyl type, bearing only one -OH function.

Advantageously, $C_2$-$C_8$ monoalcohols are non cyclic monoalcohols, still preferably they are $C_2$-$C_5$ monoalcohols and preferably $C_2$-$C_3$ monoalcohols.

The lower monoalcohols that are advantageously suitable for formulating a composition according to the present invention are those especially containing from 2 to 5 carbon atoms such as ethanol, propanol, butanol, isopropanol, isobutanol preferably ethanol and/or isopropanol and more preferably at least ethanol.

A composition of the invention may comprise at least 1% by weight, preferably at least 2 %, more preferably from 2% to 15%, advantageously from 3% to 10%, by weight and better still from 3% to 8% by weight, preferably from 4% to 6% by weight of monoalcohol(s) relative to the total weight of said composition.

There is also a need to have compositions containing changing color microcapsules in a physiological medium comprising a lower alcohol because some cosmetic ingredients are particularly soluble in hydroalcoholic media.

Furthermore, the lower monoalcohols such as ethanol allow to dissolve active agents, especially keratolytic agents, such as, for example, salicylic acid and its derivatives.

Some microcapsules of the prior art rapidly disintegrate in hydroalcoholic media, as a consequence there was a need to have compositions comprising changing color microcapsules stable in hydroalcoholic media.

## ***Polyols***

For the purposes of the present invention, the term "polyol" should be understood as meaning any organic molecule comprising at least two free hydroxyl groups. The term "polyol" according to the invention does not encompass monosaccharide-alcohol disclosed above.

Preferably, a polyol in accordance with the present invention is present in liquid form at room temperature.

The polyols/glycols are moisturizers or humectants.

They may have an effect towards the stability of other ingredients of the composition particularly towards microcapsules of the prior art.

There is thus a need to have at disposal stable compositions containing changing color microcapsules in a physiological medium comprising a polyol particularly a glycol because these compositions present a noticeable moisturizing or humecting effect.

This technical problem is solved by the compositions according to the invention. A polyol that is suitable for use in the invention may be a compound of linear, branched or cyclic, saturated or unsaturated alkyl type, bearing on each alkyl chain at least two -OH functions, in particular at least three -OH functions and more particularly at least four -OH functions.

The polyols that are advantageously suitable for formulating a composition according to the present invention are those especially containing from 2 to 32 carbon atoms preferably 2 to 20 carbon atoms and more preferably 2 to 16 carbon atoms, advantageously 2 to 10 carbon atoms, more advantageously 2 to 6 carbon atoms.

According to another embodiment, a polyol that is suitable for use in the invention may be advantageously chosen from polyethylene glycols, for example presenting 6 to 80 ethylene oxide groups.

According to one embodiment, a composition of the invention may comprise a mixture of polyols.

Advantageously, the polyol may be chosen from polyhydric alcohols, preferably of $C_2$-$C_8$ and more preferably $C_3$-$C_6$. The polyol may be chosen from glycerol, pentaerythritol, trimethylolpropane, ethylene glycol, propylene glycol, 1,3-butylene glycol, 1,3-propanediol, pentylene glycol, hexylene glycol, isoprene glycol, dipropylene glycol, diethylene glycol and diglycerol, and mixtures thereof, glycerol, sorbitol and derivatives thereof, polyglycerols, such as glycerol oligomers, for instance diglycerol, mono- and di-($C_1$-$C_5$)alkyl isosorbides such as the dimethyl isosorbide, and polyethylene glycols, glycol ethers (especially containing from 3 to 16 carbon atoms) such as mono-, di- or tripropylene glycol ($C_1$-$C_4$)alkyl ethers e.g. the dipropylene glycol methyl ether, mono-, di- or triethylene glycol ($C_1$-$C_4$)alkyl ethers e.g. diethylene glycol mono-methyl or mono-ethyl ether, and mixtures thereof.

Particularly, the polyol is selected from the group consisting in glycerol and glycols, preferably propylene glycol, butylene glycol, pentylene glycol, hexylene glycol, dipropylene glycol, diethylene glycol, ethylhexylglycerine, caprylyl glycol, glycol ethers, preferably mono-, di- or tripropylene glycol of alkyl($C_1$-$C_4$)ether or mono-, di- or triethylene glycol of alkyl($C_1$-$C_4$)ether, and mixtures thereof, more preferably the polyol is a mixture of glycerol and butylene glycol.

According to one preferred embodiment of the invention, the said polyol is chosen from ethylene glycol, pentaerythritol, trimethylolpropane, propylene glycol, butylene glycol, glycerol, polyglycerols and polyethylene glycols, and mixtures thereof.

In a particular embodiment, the polyol is selected from the group consisting in glycerol, and glycols chosen from propylene glycol, butylene glycol, ethylhexylglycerine, caprylyl glycol and mixtures thereof.

According to one particular embodiment, the composition of the invention comprises at least butylene glycol, glycerol or a mixture thereof.

In a preferred embodiment, the composition comprises at least glycerol.

According to one particular embodiment, the composition of the invention comprises glycerol as sole polyol.

Advantageously the composition may comprise from 1 to 10, preferably from 2 to 8 weight percent of glycerol based on the total weight of the composition.

Advantageously, the composition comprises from 5% to 50% by weight and in particular from 5% to 40%, and better from 6% to 30% by weight of polyol(s) based on weight of the aqueous phase.

## COSMETIC MEDIUM AND ADDITIONAL INGREDIENTS

The composition according to the invention is cosmetically acceptable, that is, it contains a physiologically acceptable medium which is non toxic and appropriate to be applied on the keratin material of human beings.

"Cosmetically acceptable" in the sense of the present invention means a composition with pleasant appearance, odor or feeling.

The "physiologically acceptable medium" is generally adapted to the form of under which the composition is intended to be conditioned.

Particularly the nature and the amount of the ingredients are adapted for example depending on whether the composition is formulated as a creamy emulsion or a fluid emulsion.

In a particular embodiment, the physiologically acceptable medium according to the invention is essentially constituted by water and comprises eventually cosmetically and dermatologically acceptable organic solvents.

The organic solvents may represent from 5% to 98% by weight relative to the total weight of the composition and may be chosen from hydrophilic organic solvents such as the water-soluble solvents described for the aqueous phase, lipophilic organic solvents, amphiphilic solvents, or mixtures thereof.

Amphiphilic organic solvents are for example derivatives of polypropylene glycol (PPG) such as the esters of PPG and fatty acids and ethers of PPG and fatty alcohols, such as the PPG-23 oleyl ether, PPG-36 oleic acid ester, and also PEG-20 methyl glucose sequistearate...

Lipophilic organic solvants are for example fatty esters such as the diisopropyl adipic acid ester, the dioctyl adipic acid ester, and alkyl benzoates.

Depending upon the form and the aim of the skin care or make-up preparation, the composition of the invention will comprise, in addition to the microcapsules containing colorant, further additional cosmetic ingredient(s) such as the ones selected from volatile and non-volatile silicon or hydrocarbon oils, surfactants, fillers, gelifying agents other than those listed above, thickening agents, emulsifiers, film forming agents, polymers, preservatives, silicone elastomere, additional non-entrapped colorants(ex: pigments, nacres...), hydrophilic or lipophilic actives such as ceramides, anti-free radical agents, insect repellent agents, slimming agents, bactericides, sequestrants, anti-dandruff agent, antioxidants, UV sunscreens, perfumes, humectants, emollients, pH regulators and mixtures thereof.

In a particular embodiment, the composition contains silicone elastomer.

Suitable silicone elastomers include, for example, emulsifying silicone elastomers such as polyglycerolated and/or hydrophilic emulsifying silicone elastomers such as alkoxylated silicone elastomers, and non-emulsifying silicone elastomers. Such silicone elastomers can be spherical or non-spherical. In a particular embodiment, the composition may comprise a non emulsifying elastomer, in particular in the form of a powder. The amount of silicone elastomer may range from 0.1 to 10% by weight of active material, in particular from 0.2 to 3%, and more preferably from 0.2 to 1% by weight of the composition.

In another particular embodiment, the composition contains UV sunscreens.

There are two groups of sunscreens: UVA sunscreens, which block UV radiation in the wavelength range of about 320 to 400 nm, and UVB sunscreens, which block radiation in the range of 290 to 320 nm. The compositions in accordance with the invention may comprise organic and/or inorganic UV sunscreen ingredients active in the UV-A and/or UV-B region which are hydrophilic and/or lipophilic.

The hydrophilic and/or lipophilic organic UV sunscreen ingredients are selected in particular from benzylidene camphor derivatives, dibenzoylmethane derivatives; cinnamic derivatives; salicylic derivatives; benzophenone derivatives; $\beta,\beta$-diphenylacrylate derivatives; p-aminobenzoic acid (PABA) derivatives; and their mixtures.

In another particular embodiment, the composition may contain nacres.

The term "nacres" should be understood as meaning lamellar pigments iridescent or non-iridescent colored particles of any form, especially produced by certain molluscs in their shell or alternatively synthesized, which have a color effect via optical interference.

The nacres may be selected from nacreous such as mica coated with an iron oxide, mica coated with bismuth oxychloride, mica coated with Titanium oxide or dioxide, mica coated with chromium oxide, mica coated with tin oxide, mica coated with $SnO_2$, mica coated by $BaSO_4$, mica coated with an organic dye and also nacreous pigments based

on bismuth oxychloride. Preferably, the nacres are white in their appearance, and they are formed preferably from mica coated with at least titanium dioxide.

As preferred nacres, we use mica coated with titanium oxide or dioxide.

In a particular embodiment, the composition contains at least one humectants.

Humectants may be chosen from polyhydric alcohols, preferably of C2-C8 and more preferably C3-C6, preferably such as glycerol, propylene glycol, 1,3-butylene glycol, pentylene glycol, hexylene glycol, dipropylene glycol, diethylene glycol and diglycerol, and mixtures thereof.

In a preferred embodiment, the composition contains at least glycerol.

The pH of the cosmetic composition according to the present invention ranges from 1 to 13, preferably from 2 to 12 and more preferably from 6 to 7.5.

It is a matter of routine operations for a person skilled in the art to adjust the nature and amount of the additives present in the compositions in accordance with the invention such that the desired cosmetic properties thereof are not thereby affected.

This make up composition, which is preferably a makeup BB product for face or a foundation, provides very strong moisturizing and freshness sensation, creamy texture with very comfortable feeling during application, and sheer natural makeup result after application. After application, all these features help to deliver a very good balance of skincare efficacy perception (creamy, moisturization and freshness) as well as makeup efficacy (proper coverage and natural radiance). Advantageously, an appropriate sunscreen agent may be added.

Otherwise, the composition may contain at least two different types of microcapsules for example three different types of microcapsules. A composition according to the invention can be obtained with pure and clean appearance of bulk, with perfect stability under -20/20°C (5 cycle), room temperature (25°C, 2 months), 37°C (2 months) and 45°C (2 months). However, capsules would release pigments during application without any particle feeling. Makeup results are perfectly and evenly provided after application. Moreover, organic sun filter can be added in the system and provide additional sun care benefit.

## Liquid fatty phase

The composition according to the invention may comprise at least one fatty phase that is liquid at room temperature and atmospheric pressure, and especially at least one oil as mentioned below.

The liquid fatty phase may further comprise one or more fatty alcohols, fatty acids such as stearic acid, or waxes such as paraffin, polyethylene wax, carnauba wax or bees wax.

Specifically, the presence of at least one oil is advantageous insofar as it may render the application of the composition easier and affords emollience.

According to the present invention, the term "oil" means a water-immiscible non-aqueous compound that is liquid at room temperature (25°C) and at atmospheric pressure (760 mmHg).

An oily phase that is suitable for preparing an anhydrous cosmetic composition according to the invention may comprise hydrocarbon-based oils, silicone oils, fluoro oils or non-fluoro oils, or mixtures thereof.

The oils may be volatile or non-volatile.

They may be of animal, plant, mineral or synthetic origin. According to one embodiment variant, oils of plant origin are preferred.

The term "volatile oil" means any non-aqueous medium that is capable of evaporating on contact with the skin or the lips in less than one hour, at room temperature and atmospheric pressure. The volatile oil is a cosmetic volatile oil, which is liquid at room temperature. More specifically, a volatile oil has an evaporation rate of between 0.01 and 200 $mg/cm^2/min$, limits inclusive.

The term "non-volatile oil" means an oil that remains on the skin or the keratin fiber at room temperature and atmospheric pressure. More specifically, a non-volatile oil has an evaporation rate strictly less than 0.01 $mg/cm^2/min$.

To measure this evaporation rate, 15 g of oil or oil mixture to be tested are placed in a crystallizing dish 7 cm in diameter, placed on a balance that is in a large chamber of about 0.3 $m^3$ which is temperature-regulated, at a temperature of 25°C, and hygrometry-regulated, at a relative humidity of 50%. The liquid is allowed to evaporate freely, without stirring it, while providing ventilation by means of a fan (Papst-Motoren, reference 8550 N, rotating at 2700 rpm) placed in a vertical position above the crystallizing dish containing said oil or said mixture, the blades being directed towards the crystallizing dish, 20 cm away from the bottom of the crystallizing dish. The mass of oil remaining in the crystallizing dish is measured at regular intervals. The evaporation rates are expressed in mg of oil evaporated per unit of area ($cm^2$) and per unit of time (minutes).

For the purposes of the present invention, the term "silicone oil" means an oil comprising at least one silicon atom, and especially at least one Si-O group.

The term "fluoro oil" means an oil comprising at least one fluorine atom.

The term "hydrocarbon-based oil" means an oil mainly containing hydrogen and carbon atoms.

The oils may optionally comprise oxygen, nitrogen, sulfur and/or phosphorus atoms, for example in the form of hydroxyl or acid radicals.

a) Volatile oils

The volatile oils may be chosen from hydrocarbon-based oils containing from 8 to 16 carbon atoms, and especially $C_8$-$C_{16}$ branched alkanes (also known as isoparaffins), for instance isododecane (also known as 2,2,4,4,6-pentamethylheptane), isodecane and isohexadecane, for instance the oils sold under the trade names Isopar® or Permethyl®, or especially linear $C_8$-$C_{14}$ alkanes.
Volatile oils that may also be used include volatile silicones, for instance volatile linear or cyclic silicone oils, especially those with a viscosity ≤ 8 centistokes (cSt) ($8 \times 10^{-6}$ $m^2/s$), and especially containing from 2 to 10 silicon atoms and in particular from 2 to 7 silicon atoms, these silicones optionally comprising alkyl or alkoxy groups containing from 1 to 10 carbon atoms. As volatile silicone oils that may be used in the invention, mention may be made especially of dimethicones with viscosities of 5 and 6 cSt, octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.
Volatile fluoro oils such as nonafluoromethoxybutane or perfluoromethylcyclopentane, and mixtures thereof, may also be used.
Advantageously, a liquid fatty phase of the invention may comprise from 1% to 50% by weight, preferably from 2% to 40% by weight and better still from 5% to 30% by weight of volatile oil(s) relative to the total weight of the said liquid fatty phase.

b) Non-volatile oils

The non-volatile oils may be chosen especially from nonvolatile hydrocarbon-based, fluoro and/or silicone oils.
Non-volatile hydrocarbon-based oils that may especially be mentioned include:

- hydrocarbon-based oils of animal origin,
- hydrocarbon-based oils of plant origin, such as phytostearyl esters, such as phytostearyl oleate, phytostearyl isostearate and lauroyl/octyldodecyl/phytostearyl glutamate (Ajinomoto, Eldew PS203), triglycerides formed from fatty acid esters of glycerol, in particular in which the fatty acids may have chain lengths ranging from $C_4$ to $C_{36}$ and especially from $C_{18}$ to $C_{36}$, these oils possibly being linear or branched, and saturated or unsaturated; these oils may especially be heptanoic or octanoic triglycerides, shea oil, alfalfa oil, poppy oil, millet oil, barley oil, rye oil, candlenut oil, passionflower oil, shea butter, aloe vera oil, sweet almond oil, peach stone oil, groundnut oil, argan oil, avocado oil, baobab oil, borage oil, broccoli oil, calendula oil, camellina oil, canola oil, carrot oil, safflower oil, flax oil, rapeseed oil, cotton oil, coconut oil, marrow seed oil, wheatgerm oil, jojoba oil, lily oil, macadamia oil, corn oil, meadowfoam oil, St John's Wort oil, monoi oil, hazelnut oil, apricot kernel oil, walnut oil, olive oil, evening primrose oil, palm oil, blackcurrant pip oil, kiwi seed oil, grapeseed oil, pistachio oil, winter squash oil, pumpkin oil, quinoa oil, musk rose oil, sesame oil, soybean oil, sunflower oil, castor oil and watermelon oil, and mixtures thereof, or alternatively caprylic/capric acid triglycerides, such as those sold by the company Stearineries Dubois or those sold under the names Miglyol 810®, 812® and 818® by the company Dynamit Nobel;
- linear or branched hydrocarbons, of mineral or synthetic origin, such as liquid paraffins and derivatives thereof, petroleum jelly, polydecenes, polybutenes, hydrogenated polyisobutene such as Parleam, Purcellin oil and squalane,
- synthetic ethers containing from 10 to 40 carbon atoms, such as dicaprylyl ether;
- synthetic esters, for instance oils of formula $R_1COOR_2$, in which $R_1$ represents a linear or branched fatty acid residue containing from 1 to 40 carbon atoms, and $R_2$ represents a hydrocarbon-based chain that is especially branched, containing from 1 to 40 carbon atoms provided that $R_1 + R_2 \geq 10$. The esters may be chosen especially from esters of alcohol and of fatty acid, for instance cetostearyl octanoate, esters of isopropyl alcohol, such as isopropyl myristate, isopropyl palmitate, ethyl palmitate, 2-ethylhexyl palmitate, isopropyl stearate, octyl stearate, hydroxylated esters, for instance isostearyl lactate, octyl hydroxystearate, alcohol or polyalcohol ricinoleates, hexyl laurate, neopentanoic acid esters, for instance isodecyl neopentanoate, isotridecyl neopentanoate, and isononanoic acid esters, for instance isononyl isononanoate and isotridecyl isononanoate.
- polyol esters and pentaerythritol esters, for instance dipentaerythrityl tetrahydroxystearate/tetraisostearate,
- esters of diol dimers and of diacid dimers, such as Lusplan DD-DA5® and Lusplan DD-DA7® sold by the company Nippon Fine Chemical and described in patent application US 2004-175 338,
- copolymers of a diol dimer and of a diacid dimer and esters thereof, such as dilinoleyl diol dimer/dilinoleic dimer copolymers and esters thereof, for instance Plandool-G,
- copolymers of polyols and of diacid dimers, and esters thereof, such as Hailuscent ISDA or the dilinoleic acid/butanediol copolymer,

- fatty alcohols that are liquid at room temperature, with a branched and/or unsaturated carbon-based chain containing from 12 to 26 carbon atoms, for instance 2-octyldodecanol, isostearyl alcohol and oleyl alcohol,
- $C_{12}$-$C_{22}$ higher fatty acids, such as oleic acid, linoleic acid or linolenic acid, and mixtures thereof,
- dialkyl carbonates, the two alkyl chains possibly being identical or different, such as dicaprylyl carbonate sold under the name Cetiol CC® by Cognis,
- oils of high molar mass, in particular with a molar mass ranging from about 400 to about 2000 g/mol and in particular from about 650 to about 1600 g/mol. As oils of high molar mass that may be used in the present invention, mention may be made especially of linear fatty acid esters with a total carbon number ranging from 35 to 70, for instance pentaerythrityl tetrapelargonate, hydroxylated esters, such as polyglyceryl-2 triisostearate, aromatic esters, such as tridecyl trimellitate, esters of branched $C_{24}$-$C_{28}$ fatty alcohols or fatty acids, such as those described in patent US 6 491 927, and pentaerythritol esters, and especially triisoarachidyl citrate, glyceryl triisostearate, glyceryl tris(2-decyl)tetradecanoate, polyglyceryl-2 tetraisostearate or pentaerythrityl tetrakis(2-decyl)tetradecanoate; phenyl silicones, such as Belsil PDM 1000 from the company Wacker (MM = 9000 g/mol), non-volatile polydimethylsiloxanes (PDMS), PDMSs comprising alkyl or alkoxy groups that are pendent and/or at the end of the silicone chain, these groups each containing from 2 to 24 carbon atoms, phenyl silicones, for instance phenyl trimethicones, phenyl dimethicones, phenyl trimethylsiloxy diphenylsiloxanes, diphenyl dimethicones, diphenyl methyldiphenyl trisiloxanes and 2-phenylethyl trimethylsiloxysilicates, dimethicones or phenyl trimethicones with a viscosity of less than or equal to 100 cSt, and mixtures thereof; and also mixtures of these various oils, and
- mixtures thereof.

According to one embodiment, the composition of the invention comprises at least one non-volatile oil chosen from non-volatile hydrocarbon-based oils such as:

- hydrocarbon-based oils of animal origin;
- hydrocarbon-based oils of plant origin;
- synthetic ethers containing from 10 to 40 carbon atoms;
- synthetic esters, for instance oils of formula $R_1COOR_2$, in which $R_1$ represents a linear or branched fatty acid residue containing from 1 to 40 carbon atoms, and $R_2$ represents a hydrocarbon-based chain that is especially branched, containing from 1 to 40 carbon atoms provided that $R_1 + R_2 \geq 10$;
- polyol esters and pentaerythritol esters;
- fatty alcohols that are liquid at room temperature, with a branched and/or unsaturated carbon-based chain containing from 12 to 26 carbon atoms;
- dialkyl carbonates, the two alkyl chains possibly being identical or different;
- oils of high molar mass; and
- mixtures thereof.

Advantageously, a liquid fatty phase of the invention may comprise at least 40% by weight, preferably at least 60% by weight or even 100% by weight of non-volatile oil(s) relative to the total weight of the said liquid fatty phase.

In another particular embodiment, the composition contains UV sunscreens.

There are two groups of sunscreens: UVA sunscreens, which block UV radiation in the wavelength range of about 320 to 400 nm, and UVB sunscreens, which block radiation in the range of 290 to 320 nm. The compositions in accordance with the invention may comprise organic and/or inorganic UV sunscreen ingredients active in the UV-A and/or UV-B region which are hydrophilic and/or lipophilic.

The hydrophilic and/or lipophilic organic UV sunscreen ingredients are selected in particular from benzylidene camphor derivatives, dibenzoylmethane derivatives; cinnamic derivatives; salicylic derivatives; benzophenone derivatives; β,β-diphenylacrylate derivatives; p-aminobenzoic acid (PABA) derivatives; and their mixtures.

## GALENIC FORMULATION

A composition according to the invention may be in the form of makeup compositions and/or care compositions for keratin materials, in particular for skin or lips. Particularly, a composition according to the invention may be a BB product or a foundation especially to be applied on the face or neck, a product for masking dark circles, a concealer product, a tinted cream, a colored composition for care or for making up the skin, especially for the face or body or an after-sun composition.

In a preferred embodiment, a composition according to the present invention is a non-rinsing composition: the composition is not intended to be rinsed after application on the skin.

In another preferred embodiment, the composition according to the present invention is not contained in a

dispenser comprising a pump. This is advantageous since it avoids the risk for the microcapsules to be broken. Indeed, when using such a dispenser, said microcapsules could be crushed before their application on the keratin materials

It is understood that the compositions according to the invention can be in any galenical form conventionally used for topical application, especially in the form of liquid or semi-liquid consistency of the milk type, or of soft, semi-solid or solid consistency of the cream or gel type, or alternatively a foam.

These compositions are prepared according to the usual methods.

The compositions of this type may be in the form of a facial and/or body care or makeup product, and may be conditioned, for example, in the form of cream in a jar or of fluid in a tube.

Preferably, the compositions of the present invention are emulsions, notably (O/W) emulsions.

In yet another preferred embodiment, the compositions of the invention are gelified compositions and in particular gelified (O/W) emulsions.

## DESCRIPTION OF DRAWINGS

Figure 1 is a schematic diagram illustrating a typical structure of a color-changing microcapsule of the present invention, wherein A represents a core and B, C, D and E being different layers concentrically surrounding said core.

Figure 2 represents a schematic diagram showing the core-shell structure of color-changing microcapsules B prepared according to Example 2 described below.

## EXAMPLES

### Microcapsules

#### Example 1: Preparation of a microcapsule A having inner brown color coating and outer white color coating

Mannitol (spray dried mannitol: Pearitol 100SD) is used as core.

To a mixed solution of 3200.0g of ethanol, 120.0g of ceramide (Ceramide PC 104) and 120.0g of hydrogenated lecithin (Lipoid S 100-3) are added and completely dissolved at 40°C. To the resulting mixture, 1260.0g of iron oxide yellow, 252.0g of iron oxide red and 45.36g of iron oxide black are added and well dispersed with a homogenizer to prepare an inner color coating solution.

347.70g of Mannitol is introduced into a fluidized bed coating system (Glatt GPCG 1, bottom spray) as a seed and subjected to a coating at 500mℓ/h of feeding rate of the inner color coating solution to obtain particles having a mannitol core coated with an inner color layer.

Thereafter, to a mixed solution of 1440.0g of ethanol, 36.0g of ceramide and 36.0g of hydrogenated lecithin are added and dissolved at 40°C. To the resulting mixture, 600.0g of titanium dioxide particles are added and well dispersed with a homogenizer to prepare a titanium dioxide particle coating solution.

A coating with the resulting titanium dioxide particle coating solution is realized by a fluidized bed process to obtain particles having an inner color layer coated with a titanium dioxide particle layer.

Then, 300.0g of shellac is dissolved in 3000g of ethanol to prepare an outer layer coating solution, which is coated onto the above titanium dioxide particle layer to obtain a color-changing microcapsule having a titanium dioxide particle layer coated with an outer layer.

#### Example 2: Preparation of a microcapsule B having inner brown color coating and outer white color coating

By using the ingredients and contents described in the below table, a color-changing microcapsule having a core and 2 layers as shown in Figure 1 is prepared by a fluidized bed process (according to a process similar to the one of example 1):

(1) Ingredients : Core seed - inner color layer - $TiO_2$ particle layer

| | | |
|---|---|---|
| **Core** | Mannitol | 13.7% |

(continued)

| | | |
|---|---|---|
| 1st layer | Sunpuro Yellow | 17.36% |
| | Sunpuro Red | 3.67% |
| | Sunpuro Black | 0.61% |
| | Lecithin | 0.20% |
| | Corn Starch Binder | 1.0% |
| 2nd layer | Titanium dioxide | qsp.100% |
| | Lecithin | 0.3% |
| | Corn Starch Binder | 1.5% |
| Percentages indicate weight percent relative to the total microcapsule weight. | | |

Compositions containing microcapsules

**Example 1 (according to invention):**

Composition in the form of a gel:

| | | **Example 1 % wgt** |
|---|---|---|
| **A1** | WATER | 21.04 |
| | PHENOXYETHANOL | 0.50 |
| | POLYSORBATE 20 | 1.00 |
| **A2** | GLYCERIN | 7.00 |
| | WATER | 1.00 |
| | IRON OXIDES | 7 |
| **B** | WATER | 21.16 |
| **C** | ISOSTEARYL NEOPENTANOATE | 9.00 |
| | AMMONIUM POLYACRYLOYLDIMETHYL TAURATE : HOSTACERIN AMPS from CLARIANT | 0.90 |
| **D** | WATER | 10.25 |
| | BUTYLENE GLYCOL | 5.00 |
| **E** | GLYCERIN (and) GLYCERYL ACRYLATE/ACRYLIC ACID COPOLYMER : NORGEL from UNITED GUARDIAN | 6.00 |
| **F** | DIMETHICONE (and) DIMETHICONOL : XIAMETER PMX-1503 FLUID from DOW CORNING | 1.00 |
| **H** | ALUMINUM STARCH OCTENYLSUCCINATE : DRY FLO PLUS from AKZO NOBEL | 3.00 |
| **I** | Microcapsule B | 3.00 |
| | **TOTAL** | **100.0** |

Protocol of preparation:

Phase A

1. Phase A1 is prepared with water at 75°C.

2. Ground phase A2 in a 3 roller mill(3 times).
3. Introduced phase A2 into phase A1.
4. Cold water (phase B) is added to the above obtained homogeneous phase for cooling to 27-30 °C.
5. Phase C (homogenized with a magnetic bar stirrer for 15-20 minutes) is introduced.
6. Phase D (homogenized with a magnetic bar stirrer) is introduced.
7. Phase E is introduced and the mixture is agitated for 10 minutes - the mixture is brought to 35 °C.
8. Phase F is introduced and the mixture is agitated for 5 minutes.
9. Phase H is introduced under slow stirring.
10. KPT microcapsules (phase I) are finally added at room temperature, applying a tooth propeller.

**Example 2 (according to invention):**

Composition in the form of an O/W emulsion:

| | | **Example 2 % wgt** |
|---|---|---|
| **A1** | WATER | 22.30 |
| | BUTYLENE GLYCOL | 7.00 |
| | | Example 2 % wgt |
| | PHENOXYETHANOL | 0.50 |
| | DISODIUM EDTA | 0.20 |
| **B1** | GLYCERYL STEARATE (and) PEG-100 STEARATE : SIMULSOL 165 from SEPPIC | 0.60 |
| | STEARIC ACID | 1.00 |
| | CETYL ALCOHOL | 0.50 |
| | C12-15 ALKYL BENZOATE | 2.00 |
| | ETHYLHEXYL METHOXYCINNAMATE | 4.00 |
| **B2** | BISMUTH OXYCHLORIDE | 0.20 |
| **C** | WATER | 11.16 |
| **D** | DIMETHICONE : XIAMETER PMX-200 SILICONE FLUID 5CS from DOW CORNING | 5.00 |
| | XANTHAN GUM | 0.25 |
| **E** | POLYACRYLAMIDE (and) C13-14 ISOPARAFFIN (and) LAURETH-7 : SEPIGEL 305 from SEPPIC | 1.00 |
| **F** | WATER | 17.00 |
| | SODIUM CITRATE | 0.72 |
| **G** | WATER | 1.00 |
| | TRIETHANOLAMINE | 0.01 |
| **H** | POTASSIUM HYDROXIDE | 0.70 |
| **I** | ALCOHOL DENAT. | 2.00 |
| **J1** | Iron oxide pigments | 7 |
| **J2** | GLYCERIN | 7.00 |
| **J3** | WATER | QS |
| **K** | Microcapsule B | 2 |
| | **TOTAL** | 100.00 |

Protocol of preparation:

1. Phase A1 is solubilized at 75°C and homogenized.
2. Phase A1 is introduced into phase B pre-heated to 75°C in a beaker, the mixture is emulsified (B1+B2) for 10 minutes.
3. Phase C is introduced.
4. Phase D (pre-mixed with a magnetic bar stirrer at about 40°C) is introduced and the mixture agitated for 10 minutes.
5. Phase E is introduced.
6. Phase F (once pre-mixed with a magnetic bar stirrer) is slowly introduced and the mixing speed is reduced
7. Phase G (pre-mixed with a magnetic bar stirrer) at 30 - 35°C is introduced, then phase H is introduced, after which phase I is added.
8. The pigments (phase J1) are ground with glycerin (phase J2) in a 3 roller mill.
9. The pigments are introduced into the beaker containing the other phases under agitation for 5 minutes.
11. Water (phase J3) is added to get 98% with gentle stirring
12. The microcapsules are introduced at the end of the preparation at room temperature (phase K) applying a tooth propeller_and weak agitation.

The compositions of examples land 2 have been compared with compositions A and B which are respectively similar to examples land 2 and wherein the Microcapsule B has been replaced by Iron oxide pigments.
The compositions of examples land 2 are satisfying in terms of make-up results. They offer better coverage, more homogeneous make-up results, ameliorated playtime and comfort than the comparative compositions.

## Claims

**1.** Color-changing composition, that is a composition which changes color upon application, for caring for and/or making up keratin materials comprising, in a physiologically acceptable medium:

a) at least 60 % by weight of an aqueous phase relative to the weight of the composition,
b) microcapsules containing releasable colorant(s),
c) at least one pigment chosen from organic pigments and non-lamellar mineral pigments, said pigment being not encapsulated in the microcapsules,
said pigment and microcapsules being present in the composition in a weight ratio of pigments / microcapsules > 1.5,

wherein the microcapsules comprise

- a core comprising one organic material, said core comprising at least one monosaccharide or monosaccharide-polyol advantageously selected from mannitol, erythritol, xylitol, sorbitol and mixtures thereof, preferably mannitol, as said organic material,
- at least one layered coating surrounding said core, the layered coating comprising at least one polymer, at least one colorant, and advantageously at least one lipid-based material,

wherein said composition comprises

- a gelifying agent chosen from polymers comprising 2-acrylamido-2-methylpropanesulphonic acid units.

**2.** Color-changing composition according to any one of the preceding claims, comprising at least 65% by weight of an aqueous phase relative to the total weight of the said composition.

**3.** Color-changing composition according to any one of the preceding claims, wherein the pigment is chosen from organic pigment chosen from synthetic or natural dyes.

**4.** Color-changing composition according to any one of the preceding claims, wherein the pigment is a non-lamellar mineral pigment, more preferably a metal oxide such as iron oxides, titanium dioxide, aluminum oxide, zirconium oxides, cobalt oxides, cerium oxides, nickel oxides, tin oxide, chromium oxide or zinc oxide, or composite oxides.

**5.** Color-changing cosmetic composition according to the preceding claim wherein the amount of 2-acrylamido-2-methylpropanesulphonic acid polymer(s) in the composition ranges, in terms of active material, from 0.01% to 15% by weight and preferably from 0.1% to 5% by weight, relative to the total weight of the composition.

**6.** Color-changing composition according to anyone of the preceding claims comprising from 0.1% to 20% by weight and preferably from 0.5% to 15% by weight of microcapsules relative to the total weight of the said composition.

**7.** Color-changing composition according to anyone of the preceding claims, wherein the layered coating surrounding said core comprises at least one hydrophilic polymer(s) selected from the group consisting of polysaccharides and derivatives, preferably the ones including one type of ose or several type of ose(s), preferably several type of ose(s) including at least D-glucose units, in particular starch and derivatives, cellulose or derivatives, and more preferably starch and derivatives.

**8.** Color-changing composition according to anyone of the preceding claims, wherein said microcapsules comprises at least:

an inner core made of monosaccharide-polyol, preferably mannitol,
at least two layers of different color,
at least one hydrophilic polymer preferably selected from polysaccharide or derivatives, and more preferably from starch or derivatives,
and advantageously at least one lipid based material, preferably an amphiphilic compound, more preferably a phospholipid, even more preferably phosphoacylglycerol such as hydrogenated lecithin.

**9.** Color-changing composition according to anyone of the preceding claims, wherein the microcapsules have a size ranging from 10 μm to 800 μm, preferably from 50 μm to 60μm in diameter of the microcapsule and comprise:

a. a core (A), preferably having a size ranging from 500 nm to 150 μm in diameter, which preferably does not contain any colorant, and comprising at least one organic core preferably selected from at least one sugar alcohol preferably a monosaccharide-polyol advantageously selected from mannitol, erythritol, xylitol, sorbitol, and mixture thereof;
b. one first layer (B) surrounding said core comprising:

i. at least one colorant, preferably iron oxide(s), and
ii. a binder selected from at least one polymer, at least one lipid-based material, and their mixture, preferably their mixture ;

c. one second layer (C) surrounding said first layer (B), preferably having a thickness of 5μm to 500μm, comprising:

i. titanium dioxide particles, and
ii. a binder selected from at least one polymer, at least one lipid-based material, and their mixture, preferably their mixture ;

d. optionally one third layer (D) surrounding said second layer (C) comprising:

i. at least one colorant, and
ii. a binder selected from at least one polymer, at least one lipid-based material, and their mixture, preferably their mixture ;

e. optionally one fourth layer (E) surrounding said third layer (D), if any, or surrounding said second layer (C) comprising

- at least one wall-forming polymer preferably selected from polysaccharides such as cellulose derivatives, in particular cellulose ether and cellulose ester, from (poly)(alkyl)(meth)acrylic acid and derivatives, notably (poly)(alkyl)(meth)acrylate and derivatives, and preferably from alkylacrylic /alkylmethacrylic acid copolymers and their derivatives.

**10.** Color-changing composition according to anyone of the preceding claims, wherein the average particle size of the

microcapsules is in the range of about 40 μm to 400 μm in diameter, preferably of about 50 μm to 300 μm in diameter, in particular from 60μm to 250μm in diameter and more preferably of about 80 μm to 200 μm in diameter.

**11.** Color-changing cosmetic composition according to anyone of the preceding claims wherein at least one layer of the microcapsules is obtained by fluid bed process.

**12.** Color-changing cosmetic composition according to anyone of the preceding claims wherein the aqueous phase comprises at least one polyol selected from the group consisting in glycerol and glycols, preferably propylene glycol, butylene glycol, pentylene glycol, hexylene glycol, dipropylene glycol, diethylene glycol, ethylhexylglycerine, caprylyl glycol, glycol ethers, preferably mono-, di- or tripropylene glycol of alkyl(C1-C4)ether or mono-, di- or triethylene glycol of alkyl(C1-C4)ether, and mixtures thereof, more preferably the polyol is a mixture of glycerol and butylene glycol.

**13.** Color-changing cosmetic composition according to anyone of the preceding claims comprising from 5% to 50% by weight and in particular from 5% to 40%, and better from 6% to 30% by weight of polyol(s) based on weight of the aqueous phase.

**14.** Color-changing cosmetic composition according to anyone of the preceding claims comprising water in an amount of at least 30% by weight, more preferably at least 40% by weight and advantageously at least 50% by weight relative to the weight of the composition.

**15.** Cosmetic process for caring for and/or making up keratinic materials, comprising application on said keratinic materials in particular on the skin of a composition as defined according to any preceding claims.

**Patentansprüche**

**1.** Farbwechselnde Zusammensetzung, die eine Zusammensetzung ist, die bei Auftrag die Farbe ändert, zum Pflegen und/oder Bilden von Keratinmaterialien, umfassend, in einem physiologisch annehmbaren Medium:

a) mindestens 60 Gew.-% einer wässrigen Phase relativ zu dem Gewicht der Zusammensetzung,
b) Mikrokapseln, die lösbare(s) Farbmittel enthalten,
c) mindestens ein Pigment, ausgewählt aus organischen Pigmenten und nicht lamellaren mineralischen Pigmenten, wobei das Pigment nicht in den Mikrokapseln eingekapselt ist,

wobei das Pigment und die Mikrokapseln in der Zusammensetzung in einem Gewichtsverhältnis von Pigmenten/Mikrokapseln > 1,5 vorhanden sind,
wobei die Mikrokapseln Folgendes umfassen:

- einen Kern, umfassend ein organisches Material, wobei der Kern mindestens ein Monosaccharid oder Monosaccharid-Polyol, vorteilhaft ausgewählt aus Mannitol, Erythrit, Xylit, Sorbitol und Gemischen davon, bevorzugt Mannitol, als ein organisches Material umfasst,
- mindestens eine geschichtete Beschichtung, die den Kern umgibt, wobei die geschichtete Beschichtung mindestens ein Polymer, mindestens ein Farbmittel und vorteilhaft mindestens ein Material auf Lipidbasis umfasst,

wobei die Zusammensetzung Folgendes umfasst:

- ein Geliermittel, ausgewählt aus Polymeren, die 2-Acrylamido-2-methylpropansulfonsäureeinheiten umfassen.

**2.** Farbwechselnde Zusammensetzung nach einem der vorhergehenden Ansprüche, umfassend mindestens 65 Gew.-% einer wässrigen Phase relativ zu dem Gesamtgewicht der Zusammensetzung.

**3.** Farbwechselnde Zusammensetzung nach einem der vorhergehenden Ansprüche, wobei das Pigment ausgewählt ist aus organischem Pigment, das aus synthetischen oder natürlichen Farbstoffen ausgewählt ist.

**4.** Farbwechselnde Zusammensetzung nach einem der vorhergehenden Ansprüche, wobei das Pigment ein nicht lamellares mineralisches Pigment ist, bevorzugter ein Metalloxid wie z. B. Eisenoxide, Titandioxid, Aluminiumoxid, Zirkonoxide, Kobaltoxide, Ceroxide, Nickeloxide, Zinnoxid, Chromoxid oder Zinkoxid oder Verbundoxide.

5. Farbwechselnde Zusammensetzung nach dem vorhergehenden Anspruch, wobei die Menge von 2-Acrylamido-2-methylpropansulfonsäurepolymer(en) in der Zusammensetzung hinsichtlich des aktiven Materials im Bereich von 0,01 Gew.-% bis 15 Gew.-% und bevorzugt von 0,1 Gew.-% bis 5 Gew.-% relativ zu dem Gesamtgewicht der Zusammensetzung ist.

6. Farbwechselnde Zusammensetzung nach einem der vorhergehenden Ansprüche, umfassend von 0,1 Gew.-% bis 20 Gew.-% und bevorzugt von 0,5 Gew.-% bis 15 Gew.-% Mikrokapseln im Verhältnis zu dem Gesamtgewicht der Zusammensetzung.

7. Farbwechselnde Zusammensetzung nach einem der vorhergehenden Ansprüche, wobei die geschichtete Beschichtung, die den Kern umgibt, mindestens ein hydrophile(s) Polymer(e) umfasst, ausgewählt aus der Gruppe bestehend aus Polysacchariden und Derivaten, bevorzugt denjenigen einschließlich einer Art -ose oder verschiedener Arten -ose(n), bevorzugt verschiedener Arten von -ose(n) einschließlich mindestens D-Glucoseeinheiten, insbesondere Stärke und Derivate, Cellulose oder Derivate, und bevorzugter Stärke und Derivate.

8. Farbwechselnde Zusammensetzung nach einem der vorhergehenden Ansprüche, wobei die Mikrokapseln mindestens Folgendes umfassen:

einen inneren Kern, der aus Monosaccharid-Polyol, bevorzugt aus Mannitol hergestellt ist,
mindestens zwei Schichten unterschiedlicher Farbe,
mindestens ein hydrophiles Polymer, bevorzugt ausgewählt aus Polysaccharid oder Derivaten und bevorzugter aus Stärke oder Derivaten,
und vorteilhaft mindestens ein Material auf Lipidbasis, bevorzugt eine amphiphile Verbindung, bevorzugter ein Phospholipid, noch bevorzugter Phosphoacylglycerin wie z. B. hydriertes Lecithin.

9. Farbwechselnde Zusammensetzung nach einem der vorhergehenden Ansprüche, wobei die Mikrokapseln eine Größe im Bereich von 10 μm bis 800 μm, bevorzugt von 50 μm bis 600 μm im Durchmesser der Mikrokapseln haben und Folgendes umfassen:

a. einen Kern (A), bevorzugt mit einer Größe im Bereich von 500 nm bis 150 μm im Durchmesser, der bevorzugt kein Farbmittel enthält, und mindestens einen organischen Kern umfasst, ausgewählt bevorzugt aus mindestens einem Zuckeralkohol, bevorzugt einem Monosaccharid-Polyol, vorteilhaft ausgewählt aus Mannitol, Erythrit, Xylit, Sorbitol und Gemischen davon;
b. eine erste Schicht (B), die den Kern umgibt, umfassend:

i. mindestens ein Farbmittel, bevorzugt Eisenoxid(e), und
ii. ein Bindemittel, ausgewählt aus mindestens einem Polymer, mindestens einem Material auf Lipidbasis und ihrem Gemisch, bevorzugt ihrem Gemisch;

c. eine zweite Schicht (C), die die erste Schicht (B) umgibt, die bevorzugt eine Dicke von 5 μm bis 500 μm hat, umfassend:

i. Titandioxidteilchen und
ii. ein Bindemittel, ausgewählt aus mindestens einem Polymer, mindestens einem Material auf Lipidbasis und ihrem Gemisch, bevorzugt ihrem Gemisch;

d. optional eine dritte Schicht (D), die die zweite Schicht (C) umgibt, umfassend:

i. mindestens ein Farbmittel und
ii. ein Bindemittel, ausgewählt aus mindestens einem Polymer, mindestens einem Material auf Lipidbasis und ihrem Gemisch, bevorzugt ihrem Gemisch;

e. optional eine vierte Schicht (E), die die dritte Schicht (D), wenn überhaupt eine, umgibt, oder die die zweite Schicht (C) umgibt, umfassend

- mindestens ein wandbildendes Polymer, bevorzugt ausgewählt aus Polysacchariden wie z. B. Cellulosederivaten, insbesondere Celluloseether und Celluloseester, aus (Poly)(alkyl)(meth)acrylsäure und Derivaten, insbesondere (Poly)(alkyl)(meth)acrylat und Derivaten, und bevorzugt aus Alkylacryl-/Alkylmethacrylsäure-Copolymeren und ihren Derivaten.

**10.** Farbwechselnde Zusammensetzung nach einem der vorhergehenden Ansprüche, wobei die durchschnittliche Teilchengröße der Mikrokapseln im Bereich von etwa 40 μm bis 400 μm Durchmesser, bevorzugt von etwa 50 μm bis 300 μm Durchmesser, insbesondere von 60 μm bis 250 μm Durchmesser und bevorzugter von etwa 80 μm bis 200 μm Durchmesser ist.

**11.** Farbwechselnde kosmetische Zusammensetzung nach einem der vorhergehenden Ansprüche, wobei mindestens eine Schicht aus den Mikrokapseln durch Fließbettverfahren erhalten werden kann.

**12.** Farbwechselnde kosmetische Zusammensetzung nach einem der vorhergehenden Ansprüche, wobei die wässrige Phase mindestens ein Polyol umfasst, ausgewählt aus der Gruppe bestehend aus Glycerin und Glycolen, bevorzugt Propylenglycol, Butylenglycol, Pentylenglycol, Hexylenglycol, Dipropylenglycol, Diethylenglycol, Ethylhexylglycerin, Caprylylglycol, Glycolethern, bevorzugt Mono-, Di- oder Tripropylenglycol aus Alkyl(C1-C4)ether oder Mono-, Di- oder Triethylenglycol aus Alkyl(C1-C4)ether und Gemischen davon, bevorzugter ist das Polyol ein Gemisch aus Glycerin und Butylenglycol.

**13.** Farbwechselnde kosmetische Zusammensetzung nach einem der vorhergehenden Ansprüche, umfassend von 5 Gew.-% bis 50 Gew.-% und insbesondere von 5 Gew.-% bis 40 Gew.-% und besser von 6 Gew.-% bis 30 Gew.-% Polyol(e) basierend auf dem Gewicht der wässrigen Phase.

**14.** Farbwechselnde kosmetische Zusammensetzung nach einem der vorhergehenden Ansprüche, umfassend Wasser in einer Menge von mindestens 30 Gew.-%, bevorzugter mindestens 40 Gew.-% und vorteilhaft mindestens 50 Gew.-% relativ zu dem Gewicht der Zusammensetzung.

**15.** Kosmetisches Verfahren zum Pflegen und/oder Bilden von Keratinmaterialien, umfassend das Auftragen auf die Keratinmaterialien insbesondere auf der Haut von einer Zusammensetzung nach einem der vorhergehenden Ansprüche.

**Revendications**

**1.** Composition changeant de couleur, qui est une composition qui change de couleur suite à son application, pour le soin et/ou le maquillage de matières kératiniques comprenant, dans un milieu physiologiquement acceptable :

a) au moins 60 % en poids, par rapport au poids de la composition, d'une phase aqueuse,
b) des microcapsules contenant un ou plusieurs colorants libérables,
c) au moins un pigment choisi parmi les pigments organiques et les pigments minéraux non lamellaires, ledit pigment n'étant pas encapsulé dans les microcapsules,

lesdits pigment et microcapsules étant présents dans la composition en un rapport en poids pigments/microcapsules > 1,5,

- un coeur comprenant un matériau organique, ledit coeur comprenant au moins un monosaccharide ou monosaccharide-polyol avantageusement choisi parmi le mannitol, l'érythritol, le xylitol, le sorbitol et leurs mélanges, de préférence le mannitol, à titre dudit matériau organique,
- au moins un revêtement stratifié entourant ledit coeur, le revêtement stratifié comprenant au moins un polymère, au moins un colorant, et avantageusement au moins un matériau à base de lipide,

laquelle composition comprend

- un agent gélifiant choisi parmi les polymères comprenant des motifs 2-acrylamido-2-méthylpropanesulfonique.

**2.** Composition changeant de couleur selon la revendication précédente, comprenant au moins 65 % en poids, par rapport au poids total de ladite composition, d'une phase aqueuse.

**3.** Composition changeant de couleur selon l'une quelconque des revendications précédentes, dans laquelle le pigment est choisi parmi les pigments organiques choisis parmi les colorants synthétiques et naturels.

**4.** Composition changeant de couleur selon l'une quelconque des revendications précédentes, dans laquelle le pigment est un pigment minéral non lamellaire, mieux encore un oxyde métallique tel que les oxydes de fer, le dioxyde de titane, l'oxyde d'aluminium, les oxydes de zirconium, les oxydes de cobalt, les oxydes de cérium, les oxydes de nickel, l'oxyde d'étain, l'oxyde de chrome ou l'oxyde de zinc, ou les oxydes composites.

**5.** Composition cosmétique changeant de couleur selon la revendication précédente, dans laquelle la quantité du ou des polymères d'acide 2-acrylamido-2-méthylpropanesulfonique dans la composition est située, en termes de matière active, dans la plage allant de 0,01 % à 15 % en poids et de préférence de 0,1 % à 5 % en poids, par rapport au poids total de la composition.

**6.** Composition changeant de couleur selon l'une quelconque des revendications précédentes, comprenant de 0,1 % à 20 % en poids et de préférence de 0,5 % à 15 % en poids, par rapport au poids total de ladite composition, de microcapsules.

**7.** Composition changeant de couleur selon l'une quelconque des revendications précédentes, dans laquelle le revêtement stratifié entourant ledit coeur comprend au moins un polymère hydrophile choisi dans l'ensemble constitué par les polysaccharides et leurs dérivés, de préférence ceux comprenant un type d'ose ou plusieurs types d'oses, de préférence plusieurs types d'oses comprenant au moins des motifs D-glucose, en particulier l'amidon et ses dérivés, la cellulose et ses dérivés, et mieux encore l'amidon et ses dérivés.

**8.** Composition changeant de couleur selon l'une quelconque des revendications précédentes, dans laquelle lesdites microcapsules comprennent au moins :

un coeur intérieur fait de monosaccharide-polyol, de préférence de mannitol,
au moins deux couches de couleur différente,
au moins un polymère hydrophile de préférence choisi parmi les polysaccharides et leurs dérivés, et mieux encore parmi l'amidon et ses dérivés,
et avantageusement au moins un matériau à base de lipide, de préférence un composé amphiphile, mieux encore un phospholipide, plus particulièrement un phosphoacylglycérol tel que la lécithine hydrogénée.

**9.** Composition changeant de couleur selon l'une quelconque des revendications précédentes, dans laquelle les microcapsules ont une taille située dans la plage allant de 10 μm à 800 μM, de préférence de 50 μm à 600 μm de diamètre de la microcapsule, et comprennent :

a. un coeur (A), ayant de préférence une taille située dans la plage allant de 500 nm à 150 μm de diamètre, qui de préférence ne contient aucun colorant, et comprenant au moins un coeur organique de préférence choisi parmi au moins un alcool de sucre, de préférence un monosaccharide-polyol avantageusement choisi parmi le mannitol, l'érythritol, le xylitol, le sorbitol, et leurs mélanges ;
b. une première couche (B) entourant ledit coeur comprenant :

i. au moins un colorant, de préférence un ou plusieurs oxydes de fer, et
ii. un liant choisi parmi au moins un polymère, au moins un matériau à base de lipide, et leurs mélanges, de préférence leurs mélanges ;

c. une deuxième couche (C) entourant ladite première couche (B), ayant de préférence une épaisseur de 5 μm à 500 μm, comprenant :

i. des particules de dioxyde de titane, et
ii. un liant choisi parmi au moins un polymère, au moins un matériau à base de lipide, et leurs mélanges, de préférence leurs mélanges ;

d. éventuellement une troisième couche (D) entourant ladite deuxième couche (C), comprenant :

i. au moins un colorant, et
ii. un liant choisi parmi au moins un polymère, au moins un matériau à base de lipide, et leurs mélanges, de préférence leurs mélanges ;

e. éventuellement une quatrième couche (E) entourant ladite troisième couche (D) éventuelle, ou entourant

ladite deuxième couche (C), comprenant

- au moins un polymère formant une paroi, de préférence choisi parmi les polysaccharides tels que les dérivés de cellulose, en particulier un éther de cellulose et un ester de cellulose, parmi les acides (poly)(alkyl)(méth)acryliques et leurs dérivés, notamment les (poly)(alkyl)méth)acrylates et leurs dérivés, et de préférence parmi les copolymères d'acide alkylacrylique/ alkylméthacrylique et leurs dérivés.

**10.** Composition changeant de couleur selon l'une quelconque des revendications précédentes, dans laquelle la granulométrie moyenne des microcapsules est située dans la plage allant d'environ 40 μm à 400 μm de diamètre, de préférence d'environ 50 μm à 300 μm de diamètre, en particulier de 60 μm à 250 μm de diamètre et mieux encore d'environ 80 μm à 200 μm de diamètre.

**11.** Composition changeant de couleur selon l'une quelconque des revendications précédentes, dans laquelle au moins une couche de microcapsules est obtenue par un procédé en lit fluidisé.

**12.** Composition changeant de couleur selon l'une quelconque des revendications précédentes, dans laquelle la phase aqueuse comprend au moins un polyol choisi dans l'ensemble constitué par le glycérol et les glycols, de préférence le propylèneglycol, le butylèneglycol, le pentylèneglycol, l'hexylèneglycol, le dipropylèneglycol, le diéthylèneglycol, l'éthylhexylglycérol, le caprylylglycol, les éthers de glycol, de préférence les éthers alkyliques en $C_1$ à $C_4$ de mono-, di- ou tri-propylèneglycol, les éthers alkyliques en $C_1$ à $C_4$ de mono-, di- ou tri-éthylèneglycol, et leurs mélanges, mieux encore le polyol est un mélange de glycérol et de butylèneglycol.

**13.** Composition changeant de couleur selon l'une quelconque des revendications précédentes, comprenant de 5 % à 50 % en poids et en particulier de 5 % à 40 % et mieux encore de 6 % à 30 % en poids, par rapport au poids de la phase aqueuse, d'un ou plusieurs polyols.

**14.** Composition changeant de couleur selon l'une quelconque des revendications précédentes, comprenant de l'eau en une quantité d'au moins 30 % en poids, mieux encore d'au moins 40 % en poids et avantageusement d'au moins 50 % en poids par rapport au poids de la composition.

**15.** Procédé cosmétique pour le soin et/ou le maquillage de matières kératiniques, comprenant l'application sur lesdites matières kératiniques, en particulier sur la peau, d'une composition telle que définie selon l'une quelconque des revendications précédentes.

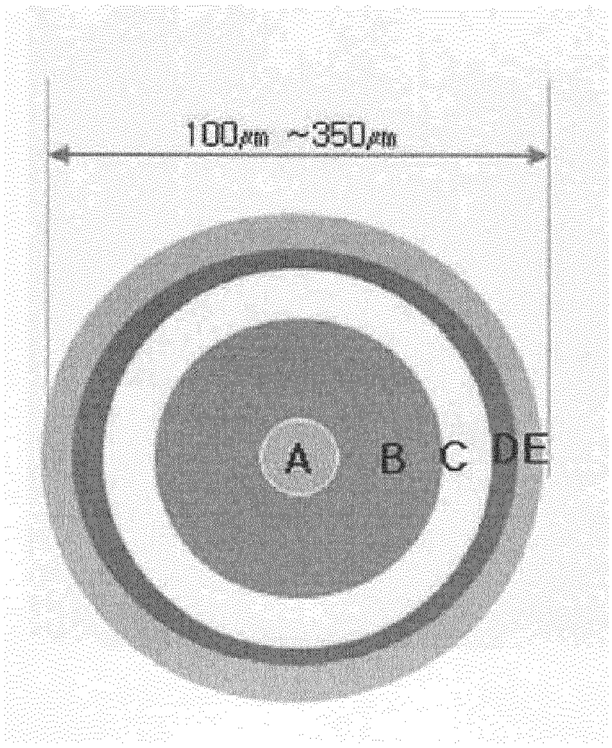


Figure 1

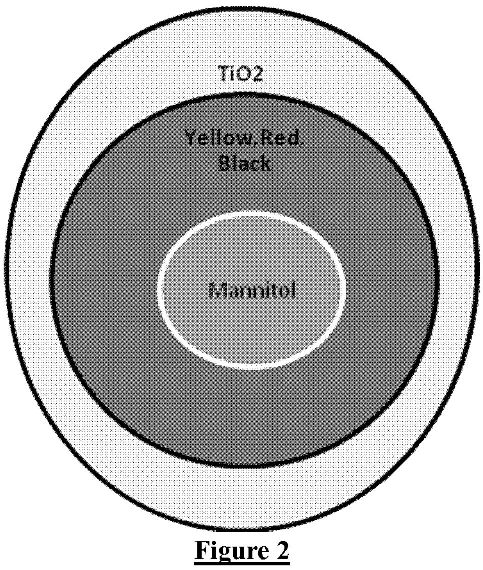


Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2011229536 A
- WO 0135933 A
- WO 2011027960 A
- FR 2841155
- WO 2008139053 A
- EP 542669 A
- EP 787730 A
- EP 787731 A
- WO 9608537 A
- US 2004175338 A
- US 6491927 B


### Non-patent literature cited in the description


- Fluid-Bed Coating, Poncelet, 2005, D. Food Science and Technology. **TEUNOU et al.** Encapsulated and Powdered Foods. United States, 2005, vol. 146, 197-212
- International Cosmetic Ingredient Dictionnary and Handbook. The Cosmetic, Toiletry, and Fragrance Association, 1997, 371-386, 524-528